(19) **Europäisches Patentamt / European Patent Office / Office européen des brevets**

(11) **EP 3 158 062 B1**

(12) **FASCICULE DE BREVET EUROPEEN**

(45) Date de publication et mention de la délivrance du brevet:
**07.08.2019  Bulletin 2019/32**

(21) Numéro de dépôt: **15732192.8**

(22) Date de dépôt: **19.06.2015**

(51) Int Cl.:
*C12N 9/10* *(2006.01)*       *C12P 19/08* *(2006.01)*
*A61K 8/73* *(2006.01)*       *A61Q 19/00* *(2006.01)*
*C08B 37/00* *(2006.01)*       *C08B 37/02* *(2006.01)*
*C08L 5/02* *(2006.01)*       *A23L 29/269* *(2016.01)*

(86) Numéro de dépôt international:
**PCT/EP2015/063864**

(87) Numéro de publication internationale:
**WO 2015/193492 (23.12.2015 Gazette 2015/51)**

(54) **DEXTRANES PRESENTANT UNE TRES HAUTE MASSE MOLAIRE**

DEXTRANE MIT SEHR HOHER MOLMASSE

VERY HIGH MOLAR MASS DEXTRANS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **20.06.2014  FR 1455751**

(43) Date de publication de la demande:
**26.04.2017  Bulletin 2017/17**

(73) Titulaires:
- **Institut National des Sciences Appliquées de Toulouse**
  **31077 Toulouse Cedex 4 (FR)**
- **Institut National de la Recherche Agronomique**
  **75015 Paris (FR)**
- **Centre National de la Recherche Scientifique**
  **75016 Paris (FR)**
- **Université de Bordeaux**
  **33000 Bordeaux (FR)**
- **Institut Polytechnique de Bordeaux**
  **33600 Pessac (FR)**

(72) Inventeurs:
- **VUILLEMIN, Marlène**
  **F-31520 Ramonville-Saint-Agne (FR)**
- **CLAVERIE, Marion**
  **F-64800 Benejacq (FR)**
- **MOULIS, Claire**
  **F-31290 Viellevigne (FR)**
- **REMAUD-SIMEON, Magali**
  **F-31520 Ramonville-Saint-Agne (FR)**
- **GRIMAUD, Florent**
  **F-31290 Villefranche de Lauragais (FR)**
- **MONSAN, Pierre**
  **F-31700 Mondonville (FR)**
- **SABATE, Agnès**
  **F-44119 Treillieres (FR)**
- **GARNIER, Catherine**
  **F-44300 Nantes (FR)**
- **DOLS-LAFARGUE, Marguerite**
  **F-33370 Fargues-Saint-Hilaire (FR)**
- **LUCAS, Patrick**
  **F-33650 Martillac (FR)**

(74) Mandataire: **Cabinet Plasseraud**
**66, rue de la Chaussée d'Antin**
**75440 Paris Cedex 09 (FR)**

(56) Documents cités:
**WO-A1-2013/017330      WO-A2-03/008618**

- **DATABASE UniProt [Online] 22 février 2012 (2012-02-22), "SubName: Full=Glucosyltransferase {ECO:0000313|EMBL:EHN59583.1};", XP002735573, extrait de EBI accession no. UNIPROT:G9WG05 Database accession no. G9WG05**

- **A. ENDO: "Oenococcus kitaharae sp. nov., a non-acidophilic and non-malolactic-fermenting oenococcus isolated from a composting distilled shochu residue", INTERNATIONAL JOURNAL OF SYSTEMATIC AND EVOLUTIONARY MICROBIOLOGY, vol. 56, no. 10, octobre 2006 (2006-10), pages 2345-2348, XP055167579, ISSN: 1466-5026, DOI: 10.1099/ijs.0.64288-0 cité dans la demande**
- **CHRISTINE RUEHMKORF ET AL: "Effect of structurally different microbial homoexopolysaccharides on the quality of gluten-free bread", EUROPEAN FOOD RESEARCH AND TECHNOLOGY ; ZEITSCHRIFT FÜR LEBENSMITTELUNTERSUCHUNG UND -FORSCHUNG A, SPRINGER, BERLIN, DE, vol. 235, no. 1, 11 mai 2012 (2012-05-11), pages 139-146, XP035071272, ISSN: 1438-2385, DOI: 10.1007/S00217-012-1746-3**
- **RIFAT Z AHMED ET AL: "Characterization of high molecular weight dextran produced byCMGDEX3", CARBOHYDRATE POLYMERS, APPLIED SCIENCE PUBLISHERS, LTD. BARKING, GB, vol. 90, no. 1, 19 mai 2012 (2012-05-19), pages 441-446, XP028432040, ISSN: 0144-8617, DOI: 10.1016/J.CARBPOL.2012.05.063 [extrait le 2012-05-28]**

Remarques:

Le dossier contient des informations techniques présentées postérieurement au dépôt de la demande et ne figurant pas dans le présent fascicule.

SEQUENCE LISTING

<110> INSA

<120> Dextranes présentant une très haute masse molaire

<130> BCT150191 QT

<160> 3

<170> PatentIn version 3.5

<210> 1
<211> 1484
<212> PRT
<213> Artificial Sequence

<220>
<223> Oenococcus kitaharae

<400> 1

```
Met Met Ala Thr Gly Ser Asn Leu Ile Thr Ala Gln Ala Asp Asp Leu
1               5               10              15

Asn Gln Glu Gly Thr Ala Ala Gln Ser Val Ser Pro Ser Thr Ala Ala
            20              25              30

Ala Asn Gln Ser Glu Ser Ser Ala Gln Ser Thr Glu Gln Ser Ala Thr
        35              40              45

Gln Ala Ala Thr Asp Gly Glu Ala Ser Thr Val Ser Thr Ala Val Thr
    50              55              60

Thr Ile Thr Pro His Tyr Val Gln Gln Ala Gly Lys Trp Leu Tyr Met
65              70              75              80

Gly Ser Asp Gly Glu Phe Val Lys Gly Pro Gln Thr Ile Asp Gly Asn
            85              90              95

Leu Gln Phe Phe Asp Glu Gln Gly Ile Gln Ile Lys Gly Ser Phe Glu
        100             105             110

Thr Val Asp Gly Ser Ser Tyr Tyr Phe Asp Ser Gln Ser Gly Asn Ala
        115             120             125

Val Thr Gly Phe Lys Ile Ile Asn Asn Asp Leu His Tyr Phe Glu Glu
    130             135             140

Asp Gly Lys Glu Thr Val Asn Asn Tyr Ala Thr Asp Lys Gln Gly Asn
145             150             155             160

Ile Phe Tyr Phe Asp Glu Asn Gly Gln Met Ala Thr Gly Val Lys Thr
```

17

|   |   |   | 165 |   |   |   |   |   | 170 |   |   |   |   | 175 |   |
|---|---|---|---|---|---|---|---|---|---|---|---|---|---|---|---|

Ile Gln Gly Gln Ser Tyr Tyr Phe Asp Gln Asp Gly His Met Arg Lys
180 185 190

Gly Tyr Ser Gly Val Phe Asp Asn Gln Val Leu Tyr Phe Asp Lys Thr
195 200 205

Thr Gly Ala Leu Ala Asn Thr Asn Val Ser Ser Ile Lys Glu Gly Leu
210 215 220

Thr Ala Gln Asn Asp Asp Phe Thr Ala His Asn Ala Val Tyr Ser Thr
225 230 235 240

Lys Ser Glu Ser Phe Thr Asn Ile Asp Gly Tyr Leu Thr Ala Glu Ala
245 250 255

Trp Tyr Arg Pro Ala Asp Ile Leu Glu Asn Gly Thr Asp Trp Arg Ala
260 265 270

Ser Arg Ala Asp Glu Phe Arg Pro Ile Leu Thr Thr Trp Trp Pro Asp
275 280 285

Lys Gln Thr Glu Val Asn Tyr Leu Asn Tyr Met Lys Thr Gln Gly Phe
290 295 300

Ile Thr Asn Asp Gln Asp Phe Lys Leu Ser Asp Asp Gln Leu Leu Leu
305 310 315 320

Asn His Ala Ala Gln Ser Val Gln Gly Glu Ile Glu Lys Lys Ile Ser
325 330 335

Gln Gln Gly Ser Thr Asp Trp Leu Lys Thr Leu Leu Gln Thr Phe Ile
340 345 350

Asn Gln Gln Pro Ser Trp Asn Gly Glu Ser Glu Asp Pro Gly Ser Asp
355 360 365

His Leu Gln Gly Gly Ala Leu Thr Phe Val Asn Ser Pro Leu Thr Pro
370 375 380

Asp Ser Asn Ser Asn Phe Arg Leu Leu Asn Arg Thr Pro Thr Asn Gln
385 390 395 400

Thr Gly Thr Pro Gln Tyr Asp Thr Asp Ala Ser Leu Gly Gly Phe Glu
405 410 415

18

```
Leu Leu Leu Ala Asn Asp Val Asp Asn Ser Asn Pro Val Val Gln Ala
        420             425             430

Glu Gln Leu Asn Trp Leu Tyr Tyr Leu Leu Asn Phe Gly Ser Ile Thr
        435             440             445

Ala Asp Asp Pro Asn Ala Asn Phe Asp Gly Ile Arg Ile Asp Ala Val
    450             455             460

Asp Asn Val Asp Ala Asp Leu Leu Gln Ile Ala Ala Ala Tyr Phe Lys
465             470             475             480

Asp Ala Phe Lys Ser Gly Ser Asn Asp Gln Thr Thr Asn Gln His Leu
            485             490             495

Ser Ile Leu Glu Asp Trp Ser His Asn Asp Pro Glu Tyr Met Lys Ala
        500             505             510

Gln Gly Tyr Pro Gln Leu Thr Met Asp Asp Tyr Met His Thr Gln Leu
        515             520             525

Ile Trp Ser Leu Thr Lys Pro Asp Asn Ile Arg Gly Thr Met Gln Arg
    530             535             540

Phe Met Asp Tyr Tyr Leu Val Asn Arg Ala Asn Asp Ser Thr Asn Asn
545             550             555             560

Glu Ala Val Ala Asn Tyr Ser Phe Val Arg Ala His Asp Ser Glu Val
            565             570             575

Gln Thr Val Ile Ala Gln Ile Ile Ser Asp Leu Tyr Pro Asn Ser Gly
        580             585             590

Ser Gly Leu Ile Pro Thr Thr Asp Gln Leu Gln Ala Ala Phe Glu Val
        595             600             605

Tyr Asn Ala Asp Met Lys Ser Asp Val Lys Lys Tyr Thr Gln Tyr Asn
        610             615             620

Ile Pro Ser Ala Tyr Ala Met Leu Leu Thr Asn Lys Asp Thr Val Pro
625             630             635             640

Arg Val Tyr Tyr Gly Asp Met Tyr Thr Asp Asp Gly Asp Tyr Met Ala
            645             650             655

Asn Lys Ser Pro Tyr Phe Asp Ala Ile Ser Thr Leu Leu Lys Ala Arg
            660             665             670
```

19

Val Lys Tyr Ala Ala Gly Gly Gln Ser Met Ala Val Asp Lys Asn Asp
    675              680            685

Ile Leu Thr Ser Val Arg Phe Gly Gln Asn Ala Met Leu Ala Ser Asp
    690              695            700

Ser Gly Asp Asn Gln Thr Arg Gln Glu Gly Ile Gly Val Ile Val Ser
705              710           715          720

Asn Asn Ser His Leu Lys Leu Ala Glu Asn Asp Gln Val Val Leu His
        725          730          735

Met Gly Ala Ala His Lys Asn Gln Ala Phe Arg Ala Leu Leu Leu Thr
        740          745          750

Ile Glu Ser Gly Leu Glu Asn Phe Asp Thr Asp Leu Gln Ala Pro Val
    755              760           765

Lys Tyr Thr Asp Ala Asn Gly Asp Leu Ile Phe Thr Ala Ala Glu Leu
    770              775           780

Ala Gly Tyr Leu Asn Pro Glu Val Ser Gly Tyr Leu Ser Ala Trp Val
785              790          795          800

Pro Val Gly Ala Ala Asp Asn Gln Asp Ala Arg Thr Ala Ala Asp Ser
        805          810          815

Ala Thr Ser Thr Asp Gly Asn Val Phe His Ser Asn Ala Ala Leu Asp
        820          825          830

Ser Asn Val Ile Phe Glu Gly Phe Ser Asn Phe Gln Ser Ile Pro Thr
        835          840          845

Ala Glu Gln His Asp Asp Phe Thr Asn Val Lys Ile Ala Glu Asn Ala
    850              855          860

Gly Leu Phe Lys Asp Trp Gly Ile Thr Ser Phe Gln Leu Ala Pro Gln
865              870          875          880

Tyr Arg Ser Ser Thr Asp Ser Thr Phe Leu Asp Ser Ile Ile Gln Asn
        885          890          895

Gly Tyr Ala Phe Thr Asp Arg Tyr Asp Leu Gly Phe Asp Thr Pro Thr
        900          905          910

Lys Tyr Gly Asp Val Asp Asp Leu Arg Ala Ala Ile Lys Ala Leu His
    915              920          925

Ala Asn Asn Ile Gln Val Met Ala Asp Trp Val Pro Asp Gln Ile Tyr
930                     935                 940

Asn Leu Gln Asn Pro Glu Ile Ile Thr Val Asn Arg Thr Asp Ser Tyr
945                 950                 955                 960

Gly Gln Pro Ile Ala Gly Ser Asp Leu Gln Asn Asp Leu Tyr Leu Ala
                965                 970                 975

Tyr Thr Asn Gly Gly Gly Gln Tyr Gln Thr Lys Phe Gly Gly Ala Phe
                980                 985                 990

Leu Glu Lys Leu Gln Gln Leu Tyr Pro Asp Leu Phe Thr Lys Thr Gln
            995                 1000                1005

Ile Ser Thr Gly Gln Thr Ile Asp Pro Ser Gln Lys Ile Thr Glu
    1010                1015                1020

Trp Ser Ala Lys Tyr Phe Asn Gly Ser Asn Ile Gln Gly Arg Gly
    1025                1030                1035

Ala Tyr Tyr Val Leu Arg Asp Ser Gly Thr Asp Gln Tyr Phe Lys
    1040                1045                1050

Val Ile Ser Asn Asp Glu Asn Glu Ala Phe Leu Pro Lys Gln Leu
    1055                1060                1065

Thr Asn Gln Pro Gly Glu Thr Gly Phe Ser Gln Asp Asp Gln Gly
    1070                1075                1080

Ile Ile Phe Phe Ser Thr Ser Gly Tyr Gln Ala Lys Asn Ala Phe
    1085                1090                1095

Val Gln Gly Asp Asp Gly Asn Tyr Tyr Tyr Phe Asp Asn Thr Gly
    1100                1105                1110

His Met Val Thr Gly Pro Gln Thr Ile Asn Gly Arg His Tyr Leu
    1115                1120                1125

Phe Phe Pro Asn Gly Val Glu Ala Gln Asn Val Phe Val Gln Asn
    1130                1135                1140

Asp Arg Gly Glu Thr Tyr Tyr Tyr Asp Gln Arg Gly Arg Gln Val
    1145                1150                1155

Ala Asn Gln Tyr Val Thr Asp Thr Asn Gly Asn Ser Phe Arg Phe

```
           1160                    1165                    1170

       Asp Glu Asn Gly Ile Met Leu Ala Asn Gln Leu Ala Gln Val Asp
           1175                1180                1185

       Gly His Trp Gln Phe Phe Lys Ser Ser Gly Val Gln Ala Lys Asp
           1190                1195                1200

       Ala Phe Ile Leu Gly Ser Asp Gly Lys Leu Arg Tyr Phe Glu Ser
           1205                1210                1215

       Gly Asn Gly Asn Met Ala Val Asn Glu Phe Lys Gly Ser Glu Asn
           1220                1225                1230

       Gly Arg Tyr Tyr Tyr Phe Gly Ala Asp Gly Gln Ala Val Ser Gly
           1235                1240                1245

       Leu Gln Thr Ile Asn Gly Arg Gln Leu Tyr Phe Asp Asp His Gly
           1250                1255                1260

       Gln Gln Met Lys Asp Ala Phe Tyr Thr Asn Gln Ser Gly Gln Arg
           1265                1270                1275

       Phe Tyr Phe Asn Ala Leu Thr Gly Asp Leu Val Lys Gly Asn Phe
           1280                1285                1290

       Ile Tyr Thr Ser Ala Ser Ser Ser Phe Thr Pro Asp Asn Asp Ser
           1295                1300                1305

       Ser Asp Ser Tyr Gln Gly Asp Ser His Leu Trp Tyr Tyr Ala Asp
           1310                1315                1320

       Ser Gln Gly Gln Ile Val Thr Gly Phe Gln Thr Ile Asn Gly His
           1325                1330                1335

       Leu Gln Tyr Phe Asp Asp Ile Ser Gly Gln Met Ile Thr Asn Arg
           1340                1345                1350

       Phe Met Arg Arg Ala Asp Gly Asn Trp Ile Tyr Leu Asp Glu Asn
           1355                1360                1365

       Gly Glu Ala Val Arg Gly Met Arg Val Ile Asn Gly Leu Thr Asn
           1370                1375                1380

       Tyr Phe Arg Asp Asp Phe Thr Gln Val Lys Asp Gly Phe Ala Gln
           1385                1390                1395
```

22

```
        Asp Pro  Asn Ser Gly Glu Arg  His Tyr Phe Asn Gly  Thr Asn Gly
            1400             1405             1410


        Ala Met  Val Thr Asn Asp Tyr  Phe Ser Pro Asp Gln  Ile His Trp
            1415             1420             1425


        Tyr Tyr  Ala Asp Asp Ser Gly  Gln Pro Val Thr Gly  Phe Gln Thr
            1430             1435             1440


        Ile Lys  Gly Gln Val Gln Tyr  Phe Asp Gln Asp Gly  Ile Gln Leu
            1445             1450             1455


        Lys Gly  Gly Ser Gln Thr Asp  Pro Val Thr Lys Gln  Thr Tyr Tyr
            1460             1465             1470


        Phe Asp  Asp Lys Phe Gly Asn  Gly Gln Ile Leu
            1475             1480
```

<210> 2
<211> 21
<212> DNA
<213> Artificial Sequence

<220>
<223> primer

<400> 2
caccatgatg gcgaccggct c        21

<210> 3
<211> 28
<212> DNA
<213> Artificial Sequence

<220>
<223> primer

<400> 3
gaggatttga ccgtttccaa acttatcg        28


**Description**

L'invention a pour objet des dextranes présentant une très haute masse molaire, tout en ayant un indice de dispersité faible.

Sauf indication contraire, on entend par « *masse molaire* » ou « *masses molaires moyennes* », dans la présente invention, la masse molaire moyenne en poids.

L'invention concerne également une dextrane-saccharase permettant de fabriquer de tels dextranes, et un procédé de fabrication desdits dextranes.

Les glucanes-saccharases d'origine bactérienne sont des transglucosylases appartenant aux familles 13 et 70 des glycoside-hydrolases. A partir de saccharose, ces enzymes catalysent la synthèse d'a-glucanes formés d'unités glucosyle. Elles peuvent également synthétiser des oligosaccharides ou glucoconjugués par réaction de transglucosylation sur des accepteurs exogènes de natures variées. Les glucanes-saccharases présentent des spécificités de produits différentes, tant au niveau des liaisons osidiques synthétisées ($\alpha$-1,2 ; $\alpha$-1,3 ; $\alpha$-1,4 ou $\alpha$-1,6) que de l'organisation de ces liaisons au sein des produits formés.

De manière générale, les glucanes-saccharases sont naturellement produites par des bactéries lactiques, par exemple des genres *Leuconostoc, Lactobacillus, Streptococcus* ou *Weissela* sp.

Parmi les glucanes-saccharases, les dextrane-saccharases produisent du dextrane, présentant de manière générale au moins 50% de liaisons osidiques $\alpha$-1,6 dans la chaîne principale, et des ramifications en $\alpha$-1,2, $\alpha$-1,3 et/ou $\alpha$-1,4. Le taux de ramifications ainsi que leur arrangement spatial varient suivant l'enzyme productrice.

Les dextranes et les dérivés de dextranes ont un nombre d'applications industrielles croissant, et dépendant de leurs tailles.

Les dextranes de masse molaire faible ou moyenne (allant généralement de $10^3$ à $7.10^4$ g.mol$^{-1}$) sont notamment utilisés pour des applications analytiques, par exemple comme supports versatiles de chromatographie, dans le domaine médical, par exemple en tant qu'extenseur du plasma sanguin grâce à leur faible caractère antigénique et leur faible viscosité en solution saline, transporteur de fer ou anticoagulant (après fonctionnalisation), dans la prévention des chocs post-opératoires, dans le traitement des brûlures ou dans la réduction de risques de thrombose ou d'embolies.

La souche *Leuconostoc mesenteroides* NRRL B-512F est un des microorganismes les plus utilisés depuis de nombreuses années pour la production industrielle de dextranes. Cette souche permet d'obtenir des dextranes de très haute masse molaire, allant de $10^6$ à plus de $10^8$ g.mol$^{-1}$. L'enzyme responsable de cette synthèse est la dextrane-saccharase appelée DSR-S. Cet homopolymère de glucose présente 95% de liaisons glucosidiques $\alpha$-1,6 et 5% de liaisons glucosidiques $\alpha$-1,3 (1). Cependant, les dextranes produits par *Leuconostoc mesenteroides* NRRL B-512F sont très polydisperses et ont un comportement rhéologique, et notamment un pouvoir viscosifiant, limitant leur utilisation en tant qu'agent texturant.

Le gène codant pour la DSR-S a pu être cloné chez *Escherichia coli* (1,2), permettant la construction de différents variants de DSR-S. Notamment, des formes tronquées au niveau des domaines N et C-terminal de DSR-S synthétisent, directement à partir du saccharose, des dextranes de masses molaires contrôlées de $4.1.10^8$, $40.10^3$ ou $1.10^3$ g.mol$^{-1}$. L'un d'entre eux, dénommé DSR-S vardel $\Delta$4N et produit sous forme recombinante par *Escherichia coli,* catalyse, à partir du saccharose, la synthèse d'un dextrane de masse moléculaire évaluée à $4.1.10^8$ g.mol$^{-1}$ et constitué de 96% de liaisons $\alpha$-1,6 et 4% de liaisons $\alpha$-1,3. La solution de dextrane obtenue avec DSR-S vardel $\Delta$4N présente une viscosité supérieure d'un facteur 10 à 100 selon la contrainte de cisaillement appliquée par rapport à celle obtenue avec l'enzyme DSR-S, et présente un comportement rhéofluidifiant. Le dextrane obtenu avec DSR-S vardel $\Delta$4N s'avère donc particulièrement intéressant pour des applications dans le domaine des agents texturants. Cependant, la viscosité de ce polymère diminue fortement lors de l'application de contraintes de cisaillement constantes de longue durée.

Les dextranes produits par les glucanes-saccharases connues jusqu'à maintenant présentent donc des inconvénients, en particulier liés à des indices de dispersité élevés ou à des masses molaires moyennes qui ne dépassent pas environ $10^8$ g.mol$^{-1}$, ainsi que des propriétés rhéologiques, notamment un pouvoir texturant, relativement limitées.

Il existe donc toujours un besoin de nouveaux dextranes dont les propriétés permettent d'élargir le champ d'applications de ce type de polymères.

Les inventeurs ont ainsi le mérite d'avoir élaboré des dextranes présentant une masse molaire extrêmement élevée, nettement supérieure à ce qui était jusqu'à présent réalisé, tout en ayant un indice de dispersité faible. Ces caractéristiques leur confèrent notamment des propriétés physico-chimiques particulièrement originales en comparaison aux dextranes jusqu'à présent synthétisés.

Les dextranes conformes à l'invention sont caractérisés par le fait qu'ils présentent entre 95% et 99%, de préférence entre 97% et 98%, de préférence encore entre 97,4% et 97.6%, de liaisons glucosidiques $\alpha$-1,6, une masse molaire moyenne en poids $M_w$ au moins égale à $0,7.10^9$ g.mol$^{-1}$, et un indice de dispersité $D_i$ compris entre 1,3 et 3.

Typiquement, pour déterminer la masse molaire moyenne en poids, on pourra utiliser la méthode décrite dans les exemples.

Selon un mode de réalisation particulier de l'invention, les dextranes conformes à l'invention présentent une

EP 3 158 062 B1

masse molaire moyenne en poids $M_w$ au moins égale à $1.10^9$ g.mol$^{-1}$. Typiquement, la masse molaire moyenne en poids $M_w$ des dextranes conformes à l'invention est inférieure à $5.10^{10}$, de préférence inférieure à $1.10^{10}$, de préférence inférieure à $5.10^9$ g.mol$^{-1}$.

Les dextranes conformes à l'invention présentent ainsi l'avantage d'avoir une masse molaire extrêmement élevée et contrôlée, de l'ordre de 10 fois supérieure aux dextranes synthétisés jusqu'à présent.

En outre, les dextranes conformes à l'invention présentent un indice de dispersité $D_i$ compris entre 1,3 et 3, de préférence compris entre 1,5 et 2,5. De préférence encore, l'indice de dispersité $D_i$ est de 1,8 ± 0,3. Ainsi, bien que leur masse molaire soit très élevée, les dextranes conformes à l'invention présentent également l'avantage d'avoir un indice de dispersité faible. En comparaison, un dextrane commercial de $2.10^6$ Da a un indice de dispersité de 3.49 (11).

On appelle ici un polymère ou un dextrane « *quasi-linéaire* », un polymère ou un dextrane dont le taux de ramification est faible et/ou dont le coefficient hydrodynamique $\upsilon_G$ est compris entre 0,44 et 0,52, de préférence compris entre 0,46 et 0,50, de préférence de 0,48. Ainsi, les dextranes conformes à l'invention présentent entre 95% et 99%, de préférence entre 97% et 98%, de préférence encore entre 97,4% et 97.6%, de liaisons glucosidiques $\alpha$-1,6. Les dextranes conformes à l'invention présentent en outre de manière avantageuse entre 1% et 5%, de préférence entre 2% et 3%, de préférence encore entre 2% et 2,5%, de liaisons glucosidiques $\alpha$-1,3. De manière particulièrement avantageuse, les dextranes conformes à l'invention présentent 97,55% de liaisons glucosidiques $\alpha$-1,6 et 2,45% de liaisons glucosidiques $\alpha$-1,3. Les dextranes conformes à l'invention sont donc quasi-linéaires.

Les caractéristiques structurales particulières des dextranes selon l'invention leur confèrent des propriétés physico-chimiques particulièrement intéressantes, permettant leur utilisation dans de nombreuses applications, et notamment dans l'industrie pétrolière, cosmétique ou dans l'agroalimentaire.

En ce qui concerne les propriétés rhéologiques, les dextranes conformes à l'invention ont une viscosité dynamique élevée pour de faibles vitesses de cisaillement et présentent un comportement de type rhéofluidifiant ou pseudo-plastique, leur viscosité dynamique diminuant avec l'augmentation du gradient de vitesse (ou vitesse de cisaillement), comme décrit à l'exemple 12.

Par ailleurs, la viscosité des dextranes conformes à l'invention est peu affectée par des contraintes de cisaillement constantes de longue durée, leur permettant d'être adaptés à des procédés industriels pour lesquels une agitation permanente est nécessaire. Par exemple, lorsque la contrainte de cisaillement est imposée à 50 s$^{-1}$ sur une durée d'environ 10 minutes, on observe une diminution de la viscosité du dextrane en solution synthétisé par DSR-S vardel $\Delta$4N, ce qui n'est pas le cas pour les dextranes conformes à l'invention, notamment synthétisés par la dextrane-saccharase DSR-OK recombinante comme mis en évidence à l'exemple 12.

En outre, les dextranes conformes à l'invention présentent un seuil d'écoulement relativement élevé, en particulier compris entre 25 et 40 Pa selon la quantité initiale de saccharose présente dans le milieu réactionnel, comme présenté à l'exemple 12.

Les dextranes conformes à l'invention ont un comportement de type gel faible. Ceci est caractérisé par des mesures de rhéologie en conditions dynamiques, avec détection des modules élastique ou module de conservation G' et le module visqueux ou module de perte G". Pour un gel, G' est supérieur à G" sur la gamme de fréquence étudiée, comme visible à l'exemple 12.

Enfin, les dextranes conformes à l'invention présentent des températures de transition vitreuse de 95°C pour un teneur en eau de 6,6%, et de 25°C pour une teneur en eau de 12,9%. En d'autres termes, pour une teneur en eau d'environ 13%, les dextranes selon l'invention présentent un état caoutchouteux, souple à des températures supérieures à 25°C environ et sont considérés comme « cassant » en dessous de 25°C environ.

La présente description concerne également une nouvelle dextrane-saccharase, appelée DSR-OK, permettant de synthétiser des dextranes conformes à l'invention.

Un objet de la présente description concerne ainsi une dextrane-saccharase dont la séquence d'acides aminés est la séquence SEQ ID NO : 1.

La séquence de l'ADN génomique de la souche *Oenococcus kitaharae* DSM 17330 a été publiée en 2012 sous la référence Genbank n°CM001398.1 (whole genome shotgun sequence) (4). Dans la base de données du NCBI, le gène codant pour DSR-OK est décrit comme Glucosyltransférase putative (accession number EHN59583).

Les inventeurs ont découvert de manière surprenante que cette séquence protéique code pour une enzyme, une dextrane-saccharase très active et particulièrement intéressante. Ce résultat était inattendu pour plusieurs raisons.

Tout d'abord, cette enzyme est issue d'une bactérie lactique, *Oenococcus kitaharae* DSM17330, isolée pour la première fois en 2006 dans des sous-produits de distillerie du shochu (3). Cette nouvelle espèce, *kitaharae*, a été identifiée à l'époque comme étant une bactérie lactique appartenant au genre *Oenococcus*, mais divergeant des *Oenococcus oeni* en étant non-acidophile et en ne réalisant pas la fermentation malolactique. Il est en outre conclu en 2006 par les chercheurs qui cultivent cette souche sur un milieu MRS, *i.e.* un milieu classique pour la croissance des bactéries lactiques, supplémenté de saccharose, que la souche *Oenococcus kitaharae* DSM17330 ne produit pas de dextrane dans le milieu extracellulaire à partir de saccharose. On aurait donc pu supposer cette Glucosyltranferase inactive sur saccharose.

Enfin, la séquence de DSR-OK ne possède qu'un maximum d'identité de 58% (sur 97% de sa séquence) avec la dextrane-saccharase DSR-N de *Leuconostoc mesenteroides* KIBGE IB-22 dont la séquence est disponible dans les bases de données sous le numéro d'accession Genbank AFP53921.1. Comparé à d'autres séquences putatives de glucane-saccharases pouvant être identifiées suite à des campagnes de séquençage de génomes bactériens, ce pourcentage d'identité est faible. Par ailleurs, la base de données CAZy, recensant l'ensemble des enzymes actives sur les sucres, ne fait encore aucune mention de cette souche et de ce gène dans la famille GH-70, famille des glucane-saccharases.

L'homme du métier n'était donc pas amené à considérer cette enzyme putative comme une potentielle dextrane-saccharase intéressante par ses propriétés catalytiques et les dextranes synthétisés.

Les propriétés exceptionnelles de DSR-OK en tant que dextrane-saccharase ont finalement été mises en évidence par les inventeurs.

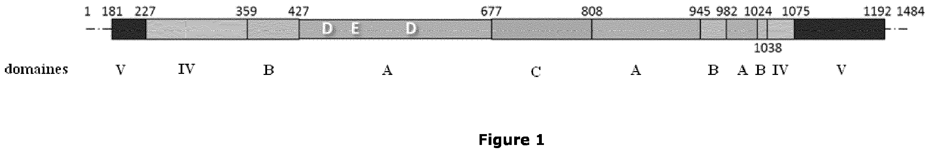

La dextrane-saccharase DSR-OK est composée de 1484 acides aminés, possédant la triade catalytique DED et les 4 motifs conservés décrits habituellement chez les enzymes de la famille 70 des glycoside-hydrolases. Les motifs protéiques conservés du coeur catalytique (I à IV) ont ainsi été identifiés de la position 936 à la position 942 pour le motif I, de la position 458 à la position 468 pour le motif II, de la position 495 à la position 505 pour le motif III et de la position 568 à la position 582 pour le motif IV. En comparaison avec la séquence protéique de la glucane-saccharase GTF-180, les cinq domaines structuraux classiquement décrits pour les glucane-saccharases (A, B, C, IV et V) (5) peuvent être identifiés dans la structure primaire de DSR-OK (figure 1).

DSR-OK est une dextrane-saccharase très spécifique de la polymérisation par liaisons osidiques de type $\alpha$-1,6, et est une excellente polymérase. En effet, comme visible à l'exemple 5, les analyses chromatographiques réalisées suite à une synthèse de dextrane à partir de 100 g.L$^{-1}$ de saccharose montrent qu'environ 89% des unités glucosyle issues du substrat sont utilisées pour la production du polymère.

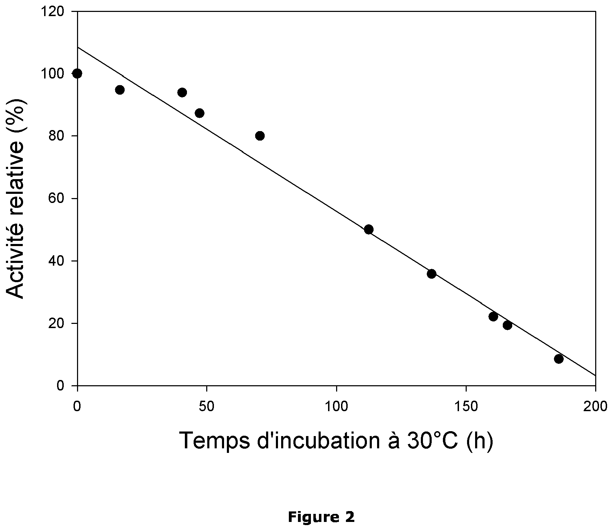

En outre, DSR-OK a une température optimale de fonctionnement d'environ 30 °C et un pH optimal d'environ 5,75. A 30 °C, cette dextrane-saccharase est stable et robuste, son temps de demi-vie étant d'environ 111 h lors d'une caractérisation en milieu brut, *i.e.* non purifié, comme visible à la figure 2. Cette propriété est particulièrement intéressante et rare chez les glucane-saccharases. Ainsi, en comparaison, DSR-S vardel Δ4N, autre glucane-saccharase recombinante, présente un temps de demi-vie de seulement 24h dans les mêmes conditions.

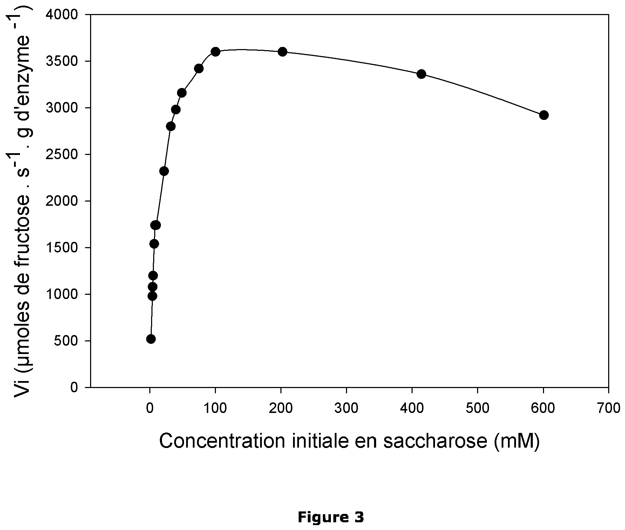

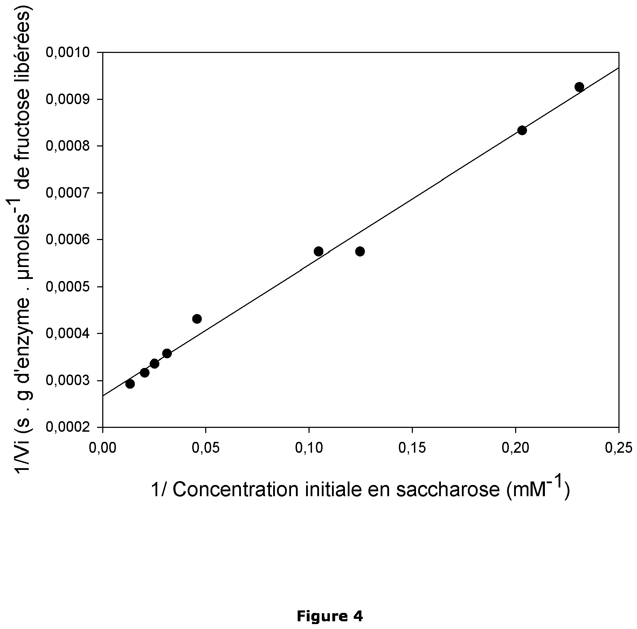

Par ailleurs, DSR-OK suit un mécanisme Michaelien, avec une inhibition par excès de substrat, et est un catalyseur très performant en comparaison à d'autres dextrane-saccharases similaires, comme présenté à l'exemple 8. Par exemple, DSR-OK a une constante d'affinité, Km, de 8.99 mM et une constante catalytique, Kcat ,de 550 s$^{-1}$ (voir figures 3 et 4). L'enzyme a donc une bonne affinité pour le substrat (Km), du même ordre de grandeur que la plupart des dextrane-saccharases caractérisées, et présente une constante catalytique (kcat) excellente, rarement observée chez les glucane-saccharases. L'enzyme est donc extrêmement efficace pour catalyser des réactions de polymérisation à partir de saccharose.

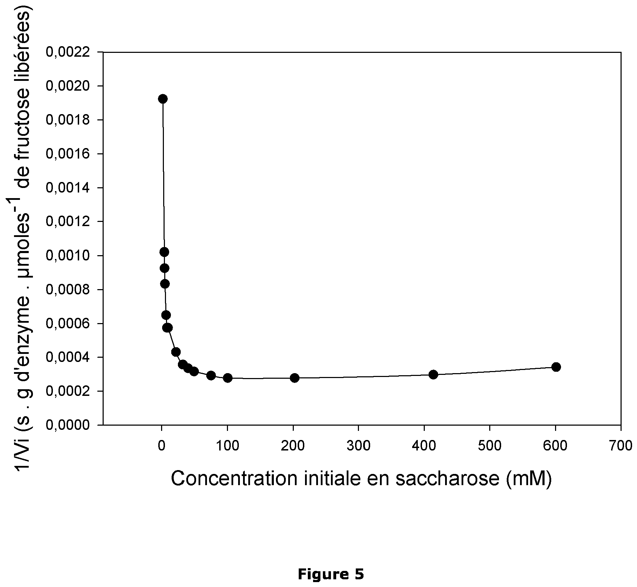

Enfin, comme visible à la figure 5, la constante d'inhibition de DSR-OK s'élève à 1M, ce qui montre que cette dextrane-saccharase est peu inhibée par un excès de substrat. Cette caractéristique permet son utilisation dans des procédés de synthèses de dextrane à partir de concentrations très variables en saccharose initial selon la masse moléculaire du dextrane visé. En effet, il est connu que l'augmentation de la concentration en substrat initial favorise la synthèse de dextranes de masses moléculaires plus faibles (2).

Les variants conservateurs de fonction sont également compris dans la présente demande. On appelle « *variant conservateur de fonction* », un variant dans lequel un (ou plusieurs) résidu d'acide aminé donné dans une protéine a été modifié sans pour autant altérer la conformation globale et l'activité enzymatique de dextrane-saccharase permettant de synthétiser des dextranes conformes à l'invention. Typiquement, la modification peut concerner des parties non sensibles de l'enzyme, et ne concerne notamment pas la triade catalytique.

Ainsi, un mode de réalisation particulier de l'invention concerne les variants conservateurs de fonction. Typiquement, un tel variant conservateur de fonction est une dextrane-saccharase dont la séquence d'acides aminés a au moins 80%, de préférence 85%, de préférence encore 90%, de préférence encore 95%, de préférence encore 98% d'identité sur les positions allant de 359 à 1038 sur la séquence d'acides aminés SEQ ID NO : 1, ce qui correspond aux domaines A+B+C de l'enzyme, à condition que l'activité enzymatique de dextrane-saccharase, permettant de synthétiser des dextranes conformes à l'invention, soit maintenue.

Une dextrane-saccharase selon l'invention est un variant de l'enzyme dextrane-saccharase de séquence SEQ ID NO : 1, à condition que ce variant soit conservateur de fonction de l'enzyme.

Un objet de l'invention se rapporte ainsi à une dextrane-saccharase ayant au moins 80%, de préférence 85%, de préférence encore 90%, de préférence encore 95%, de préférence encore 98% d'identité avec la séquence allant des positions 359 à 1038 de la séquence d'acides aminés SEQ ID NO : 1, la dextrane-saccharase n'ayant pas pour séquence d'acides aminés la séquence SEQ ID NO : 1.

L'activité enzymatique d'un variant de dextrane-saccharase peut être testée selon des techniques connues de l'homme du métier, par exemple par la méthode à l'acide dinitrosalicylique (DNS) de Sumner et Howell (10) ou par analyse HPLC.

Selon un mode de réalisation particulier, une dextrane-saccharase selon l'invention peut être préparée par des techniques connues de recombinaison génétique.

L'homme du métier saura utiliser les technologies de biologie moléculaire et saura choisir un système d'expression adapté selon les techniques qui lui sont connues. On peut ici se référer à l'exemple 2 ci-après.

Le terme *"système d'expression"* comprend une cellule hôte et un vecteur compatible dans des conditions appropriées, c'est-à-dire des conditions permettant l'expression de la protéine codée par l'ADN étranger porté par le vecteur et introduit dans la cellule hôte. Typiquement, la séquence d'acides nucléiques codant une dextrane-saccharase, de préférence une dextrane-saccharase DSR-OK telle que précédemment décrite ou un variant conservateur de fonction, peut être insérée dans un vecteur d'expression approprié qui sera alors introduit dans une cellule hôte procaryote ou eucaryote adéquat.

Un vecteur d'expression est typiquement un plasmide, un cosmide, un épisome, un chromosome artificiel, un phage ou un vecteur viral.

Typiquement, l'expression des acides nucléiques de la présente invention peut être réalisée dans des cellules hôtes procaryotes ou eucaryotes. Comme exemple non limitatifs de souches de cellule hôte procaryotes, on peut citer des souches telles que *Escherichia coli, Bacillus subtilis, Salmonella typhimurium* ou des souches du genre *Pseudomonas, Streptomyces* et *Staphylococcus.* Comme exemple non limitatifs de souches de cellules hôtes eucaryotes, on peut citer des souches telles que des parasites *Apicomplexan (Plasmodia, Toxoplasma, Cryptosporidia) Leishmania* ou *Trypanosoma*, ou des cellules de levure telles que *Saccharomyces* comme *Saccharomyces cerevisiae* ou *pombe*, *Pichia pastoris* etc.

De manière préférentielle, des cellules hôtes procaryotes sont utilisées. Selon un mode de réalisation avantageux, les cellules utilisées pour l'expression des acides nucléiques de la présente invention sont *Escherichia coli* et de manière encore préférentielle, les souches sont choisies parmi TOP10, BL21AI, BL21 Star DE3, Arctic Express DE3.

De manière avantageuse, la dextrane-saccharase peut comprendre en outre, à l'extrémité N-terminale, une séquence signal. Cette séquence signal peut être une des séquences signal connues de l'homme du métier, notamment afin que, lorsque la protéine est synthétisée dans une cellule hôte, la synthèse de dextrane puisse être effectuée de manière extracellulaire. En effet, la dextrane-saccharase DSR-OK ne possède pas de peptide signal. La présence d'un peptide signal adapté permet ainsi la synthèse de dextrane de manière extracellulaire. De plus, des outils génétiques sont de plus en plus développés pour l'expression hétérologue chez des microorganismes GRAS (Generally Recognized As Safe) du genre *Bacillus* et *Lactococcus.* Ainsi, il existe à l'heure actuelle, plusieurs systèmes connus d'excrétion de protéines recombinantes chez ces bactéries, pouvant être utilisées dans le cas présent par l'homme du métier.

Comme mentionné à l'exemple 2, la production recombinante de dextrane-saccharase chez *Escherichia coli* utilisant les conditions décrites dans l'exemple 2 atteint environ 30 000 Unités d'activité enzymatique par litre de culture, ce qui permet son utilisation à faible coût dans des procédés de synthèse de polymère.

Une dextrane-saccharase selon l'invention peut ainsi être préparée par culture de cellules hôtes contenant une séquence d'acides nucléiques codant une dextrane-saccharase, de préférence une dextrane-saccharase DSR-OK telle que précédemment décrite ou un variant conservateur de fonction, dans des conditions permettant l'expression d'une dextrane-saccharase, et par isolement de ladite dextrane-saccharase du milieu de culture selon les techniques connues de l'homme du métier.

De telles dextrane-saccharases peuvent ensuite être purifiées par toute technique de purification connue de l'homme du métier, par exemple par précipitation, chromatographie par échange d'ions, chromatographie d'affinité, chromatographie d'échange hydrophobe, filtration sur gel, chromatographie HPLC en phase inverse, etc. Selon un mode de réalisation préférentiel, la dextrane-saccharase obtenue par culture de cellule hôte est purifiée par chromatographie d'affinité. Elles peuvent également être immobilisées par des techniques connues de l'homme du métier, notamment par exemple par adsorption, formation de liaison covalente entre le support et la protéine, inclusion ou encapsulation.

Selon un autre mode de réalisation, une dextrane-saccharase selon l'invention peut être préparée directement par culture de la souche native *Oenococcus kitaharae.* La souche *Oenococcus kitaharae* ne possède en effet qu'un seul gène de dextrane-saccharase, codant pour DSR-OK.

L'invention concerne aussi la fabrication de dextranes conformes à l'invention.

Un objet de l'invention concerne ainsi un procédé de fabrication de dextranes conformes à l'invention comprenant la réaction d'une dextrane-saccharase de séquence SEQ ID NO : 1, ou un variant conservateur de fonction tel que précédemment décrit, avec du saccharose.

L'homme du métier saura adapter les conditions de fermentation à appliquer pour avoir une production optimisée de dextrane.

D'une manière générale, la dextrane-saccharase doit être incubée dans un milieu de synthèse contenant du saccharose.

Selon un mode de réalisation particulier, des microorganismes secrétant de la dextrane-saccharase ou des extraits cellulaires de microorganismes produisant de la dextrane-saccharase de manière intracellulaire peuvent être cultivés ou utilisés dans un milieu comportant du saccharose, ceci menant à la synthèse de dextrane conforme à l'invention.

Comme précédemment décrit, la synthèse de dextrane peut être réalisée à partir de dextrane-saccharase native ou de dextrane-saccharase recombinante, purifiée ou non.

Ainsi, selon un autre mode de réalisation particulier, une dextrane-saccharase recombinante, de préférence la dextrane-saccharase DSR-OK ou un variant conservateur de fonction, est incubée dans un milieu de synthèse contenant du saccharose.

De manière avantageuse, la réaction est conduite à une température comprise entre 20°C et 40°C, de préférence comprise entre 25°C et 35°C, de préférence encore comprise entre 30°C et 33°C. Typiquement, la réaction est conduite à une température d'environ 30°C.

L'homme du métier saura adapter la durée de la synthèse, en particulier en fonction de la quantité d'enzyme ajoutée dans le milieu réactionnel et en fonction de la température. Typiquement, la réaction est conduite pendant une durée comprise entre 4 heures et 20 heures, de préférence comprise entre 8 heures et 16 heures, de préférence pendant une durée d'environ 12 heures.

De plus, la concentration en saccharose est de préférence comprise entre environ 50 et 200 g.L$^{-1}$, de préférence entre environ 50 et 150 g.L$^{-1}$. Typiquement, la concentration en saccharose est d'environ 100 g.L$^{-1}$. De manière générale, il est connu que la masse molaire moyenne du dextrane synthétisé sera plus susceptible d'être élevée en utilisant une concentration initiale en saccharose faible.

De manière avantageuse, le pH lors de la réaction est compris entre environ 5 et 6,5, de préférence entre 5 et 6, de préférence encore entre 5 et 5,8. Typiquement, le pH lors de la réaction est d'environ 5,75.

Un objet de l'invention concerne une composition cosmétique ou alimentaire comprenant des dextranes tels que précédemment décrits et au moins un support pharmaceutiquement acceptable ou alimentaire.

Des dextranes conformes à l'invention pourront être utilisés dans tout type d'application pour lesquelles ses propriétés physico-chimiques sont adaptées, par exemple des applications nécessitant des dextranes de haute masse molaire et/ou une viscosité importante à faible cisaillement, un comportement rhéofluidifiant, avec une viscosité stable dans le temps lors de contraintes de cisaillement constantes de longue durée, et présentant un seuil relativement élevé d'écoulement (de l'ordre de 30 Pa).

Des dextranes conformes à l'invention peuvent ainsi être utilisés dans l'industrie pétrolière, cosmétique ou encore dans l'industrie agroalimentaire.

A titre d'exemples non limitatifs, des dextranes conformes à l'invention peuvent être utilisés pour la récupération assistée du pétrole. Ils peuvent aussi être utilisés dans les domaines du forage et des mines, par exemple en tant qu'agent floculant, comme décrit dans la demande FR 2 978 773 A. Des dextranes conformes à l'invention peuvent également être utilisés comme agents épaississants, gélifiants et/ou texturants, par exemple comme substitut de gomme arabique, ou dans des produits comme les produits gélifiés, les sauces, pour des produits de boulangerie, confiserie ou pour les crèmes glacées. On peut par exemple se référer au brevet US 6,627,235 qui décrit l'utilisation de dextranes de haute masse molaire dans les produits de boulangerie pour améliorer la texture des produits, ainsi que leur résistance à la cuisson et au rassissement.

Un objet de l'invention concerne ainsi l'utilisation de dextranes conformes à l'invention en tant qu'agent épaississant, gélifiant et/ou texturant.

L'invention concerne également des hydrolysats de dextranes.

On entend ici par « *hydrolysat de dextrane* », un dextrane conforme à l'invention et subissant une ou plusieurs étapes de traitement supplémentaires aptes à lui conférer une masse molaire contrôlée inférieure à la masse molaire atteinte selon la présente invention. De telles méthodes sont connues de l'homme du métier. Par exemple, une hydrolyse acide suivie d'un fractionnement, notamment au moyen de solvants organiques, peuvent être appliqués.

L'invention concerne également des réactions d'accepteur réalisées à partir de saccharose et de gluco-oligosaccharides rajoutés au milieu réactionnel en début de synthèse (glucose ou maltose, par exemple). Cette réaction d'accepteur est bien connue de l'homme du métier pour favoriser la synthèse de dextranes de plus faibles masses molaires qu'à partir de saccharose seul, et les produits obtenus peuvent avoir des applications similaires à celles des dextranes obtenus par hydrolysats.

Les dextranes obtenus, de taille inférieure, peuvent ainsi être utilisés dans tous les domaines pour lesquels une telle masse molaire plus faible est appropriée, par exemple pour des applications pharmaceutiques (substitut de plasma sanguin, transporteur de fer ou anti-coagulant), pour des applications analytiques (supports versatiles de chromatographie), dans le domaine médical (extenseur du plasma sanguin) etc. On peut ainsi se référer à la publication Vettori et al. (6).

Un objet de l'invention concerne un procédé de fabrication d'un hydrolysat de dextrane, dans lequel un dextrane conforme à l'invention subit des étapes d'hydrolyse et de fractionnement.

L'invention concerne enfin des dérivés de dextranes.

On entend ici par « *dérivé de dextrane* », un dextrane conforme à l'invention et subissant une ou plusieurs étapes de modification chimiques connues, notamment choisies parmi une éthérification, une estérification ou une réticulation. On peut ainsi obtenir un dérivé de dextrane tel qu'un dextrane réticulé, un ester de dextrane, par exemple un ester inorganique de dextrane (phosphate de dextrane, sulfate de dextrane) ou un ester organique de dextrane, un éther de dextrane, par exemple un éther de dextrane non-ionique (dextrane alkylé, éther d'hydroxyalkyle ou éther hydroxyalkyle aryle de dextrine, éther de dextrane poly(éthylène-glycol)-alkyle) ou un éther de dextrane ionique (dextrane sulfopropylé, dextrane carboxyméthylé, dextrane 2-(Diéthylamino)éthyl). De telles techniques de modification chimiques, ainsi que les applications pour lesquelles les dextranes ainsi obtenus sont adaptés, sont bien connues de l'homme du métier. On peut par exemple se référer au document Heinze et al (7) et à la thèse de Ndegwa Henry Maina (8).

Un objet de l'invention se rapporte donc à un procédé de modification d'un dextrane conforme à l'invention, dans lequel le dextrane subit une ou plusieurs étapes de modification chimique.

De manière avantageuse, une étape de modification chimique est choisie parmi une éthérification, une estérification et une réticulation.

## FIGURES

**Figure 1:** Représentation schématique de la structure primaire de DSR-OK (basée sur l'alignement protéique avec l'enzyme GFT180 de *Lactobacillus reuteri* 180). Cinq domaines sont distincts : domaine V en rouge, domaine IV en jaune, domaine B en vert, domaine A en bleu et domaine C en violet. La triade catalytique est indiquée par les lettres blanches « D », « E » et « D ».

**Figure 2** : Représentation graphique de l'activité relative de la dextrane-saccharase DSR-OK à 30°C en fonction du temps.

**Figure 3** : Détermination de la constante d'inhibition par excès de substrat (variation de l'inverse de la vitesse initiale en fonction de la concentration initiale en substrat).

**Figure 4** : Représentation graphique de Michaelis-Menten (variation de la vitesse initiale de production de fructose en fonction de la concentration initiale en substrat).

**Figure 5** : Représentation graphique de Linweaver-Burk (variation de l'inverse de la vitesse en fonction de l'inverse de la concentration en substrat).

**Figure 6** : Spectre obtenu par RMN du proton sur le dextrane synthétisé par DSR-OK recombinante. Les flèches correspondent aux liaisons $\alpha$-1,3 et $\alpha$-1,6.

**Figure 7** : Profil d'élution obtenu par AF4-MALLS (Asymmetric Flow-Field-Flow-Fractionation - Multi-Angle Laser Light Scattering) (D1 = réponse réfractométrique, D1-DL = diffusion de lumière) et distribution en masse molaire du dextrane produit par préparation de DSR-OK recombinante (D1 Mw).

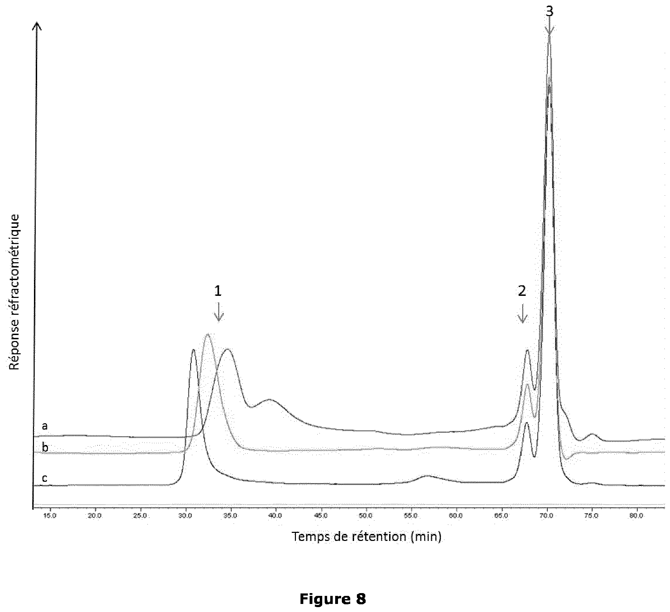

**Figure 8** : Profils d'élution obtenus par HPSEC des dextranes produits à partir de 100 g.L$^{-1}$ de saccharose par (a) DSR-S native produite par L. mesenteroides NRRL B-512F, (b) l'enzyme recombinante DSR-S vardel Δ4N et par (c) l'enzyme recombinante DSR-OK. Le pic 1 correspond au dextrane de très haute masse molaire, le pic 2 à des oligosaccharides de degré de polymérisation inférieur à 7, et le pic 3 correspond au fructose co-produit lors de la réaction.

**Figure 9** : Représentation du rayon de giration (en nm) en fonction de la masse molaire pour différents dextranes : gcn1 produit par un mutant de DSR-S vardel Δ4N, le dextrane produit par DSR-OK et un dextrane commercial T2000 (Pharmacosmos) de 2.10$^6$ g.mol$^{-1}$.

**Figure 10** : Comportement rhéologique du dextrane en fonction de la concentration en saccharose par mesure de la viscosité du dextrane synthétisé par DSR-OK dans le milieu de synthèse brut à partir de différentes concentration en saccharose : 50 g.L$^{-1}$ (●), 75 g.L$^{-1}$ (▲), 100 g.L$^{-1}$ (■) et 150 g.L$^{-1}$ (○).

**Figure 11** : Comportement rhéologique du dextrane purifié par mesure de la viscosité du dextrane synthétisé par DSR-OK, purifié par précipitation à l'éthanol, et concentré à 50 g.L$^{-1}$ dans de l'eau distillée.

**Figure 12 :** Mesure du seuil d'écoulement du dextrane produit par DSR-OK dans le milieu de synthèse brut, à partir de 100 g.L$^{-1}$ de saccharose par représentation graphique du taux de cisaillement du dextrane à 33 g.L$^{-1}$ produit par DSR-OK en fonction de la contrainte de cisaillement appliquée.

**Figure 13 :** Comportement rhéologique du dextrane produit par DSR-OK par mesure des modules G' (•) et G" (○) par balayage en fréquences en conditions oscillatoires (oscillations entre 10$^{-2}$ et 10$^{2}$ rad.s$^{-1}$) : comportement de type gel G'>G".

**Figure 14** : Comportement rhéologique de différents dextrane par mesure de la viscosité en fonction de la contrainte de cisaillement de dextrane produit en présence de 100 g.L$^{-1}$ de saccharose, par la DSR-OK recombinante (■), par la DSR-S native issue de *L. mesenteroides* NRRL B-512F (-), par la DSR-S vardel D4N (◊).

**Figure 15** : Evolution de la viscosité apparente des dextranes dans leur milieu de synthèse brut synthétisés à partir de 100 g.L$^{-1}$ de saccharose A : par DSR-OK recombinante et B : par DSR-S vardel D4N à contrainte de cisaillement constante de 50 s$^{-1}$, à 20°C et pression atmosphérique ambiante.

**EXEMPLES**

**Exemple 1 : Identification du gène *dsrok* dans le génome *Oenococcus kitaharae* DSM 17330 et analyse de la structure primaire de la protéine correspondante**

Le gène *dsrok* a été identifié au sein du génome d'*Oenococcus kitaharae* DSM 17330 (disponible dans la base de données NCBI sous le numéro de référence NZ_CM001398) par blast nucléotidique contre une base de données constituée de séquences nucléotidiques de glucane-saccharases répertoriées dans la famille des glycoside-hydrolases 70 selon CAZY (Carbohydrate Active enZYme database, www.cazy.org/GH70_all.html).
Le gène a été traduit en séquence protéique grâce au logiciel disponible en ligne, Transeq d'EMBOSS (www.ebi.ac.uk/Tools/st/emboss_transeq/).
L'absence de peptide signal a été prédite par le logiciel SignalP server 4.1 (www.cbs.dtu.dk/services/SignalP/).
Des alignements protéiques multiples (avec le logiciel d'alignement global, ClustalW2, disponible en ligne, www.ebi.ac.uk/Tools/msa/clustalw2/) avec d'autres glucane-saccharases caractérisées ont permis d'identifier les motifs conservés du coeur catalytique de DSR-OK, et de découper l'enzyme en différents domaines protéiques (A, B, C, IV et V).
Les différents pourcentages d'identité et de similarité entre séquences protéiques, indiqués dans la fiche préliminaire d'invention, ont été calculés avec l'outil BlastP (protein-protein Blast) de NCBI, disponible en ligne et en utilisant les paramètres par défaut proposés par le site.

**Exemple 2 : Clonage et expression hétérologue de la protéine chez *Escherichia coli***

Le gène *dsrok* a tout d'abord été amplifié par PCR, à partir de l'ADN génomique de la souche *Oenococcus kitaharae* DSM 17330, en utilisant les deux amorces présentés dans le tableau 1.

*Tableau 1*

| Amorce | Séquence (5' à 3') |
|---|---|
| amorce Forward | CACCATGATGGCGACCGGCTC (SEQ ID NO :2) |
| amorce Reverse | GAGGATTTGACCGTTTCCAAACTTATCG (SEQ ID NO :3) |

L'addition des 4 bases, CACC, en 5' de l'amorce forward a permis l'insertion correcte du fragment PCR dans le vecteur d'entrée pENTR/D/TOPO (Life Technologies), afin de procéder par la suite à un clonage utilisant la technologie Gateway.
Un clone d'entrée positif (vecteur d'entrée contenant le fragment PCR dans le sens désiré) a été sélectionné et recombiné avec le vecteur de destination pET-53-DEST (Novagen) à l'aide de la LR clonase enzyme mix II (Life technologies). Les clones recombinants positifs ont été sélectionnés et analysés par restriction. L'absence de mutation au sein des plasmides a été confirmée par séquençage (GATC).
Pour la production de l'enzyme recombinante, des cellules d'*Escherichia coli* BL21 star DE3 ont été transformées avec le plasmide pET-53/DSR-OK construit comme indiqué précédemment. 300 μL du mix de transformation ont été ensemencés dans 30 mL de milieu LB (Lysogeny Broth), supplémenté avec 100 μg.mL-1 d'ampicilline, et mis à incuber pendant la nuit à 37 °C afin de préparer une préculture.

Des cultures d'IL en milieu ZYM5052 modifié (1% glycérol, 0% glucose, 1% lactose, Studier, 2005) ont ainsi été ensemencées à une densité optique D0600 nm initiale de 0,05 à partir de la préculture de la veille, puis mises à incuber 24 heures à 23 °C et 150 rpm. En fin de fermentation, les milieux de culture sont centrifugés (15 min, 6500 rpm, 4 °C) et les culots sont concentrés à une DO de 80 dans 50 mM de tampon acétate de sodium, pH 5,75.

Afin d'obtenir l'enzyme recombinante (produite par *Escherichia coli* de manière intracellulaire), les cellules sont cassées aux ultrasons selon le protocole suivant : 5 cycles de 20 secondes à 30% de la puissance maximale de la sonde, à froid, espacés de 4 minutes de repos dans la glace. Le surnageant de sonication (contenant l'enzyme recombinante soluble) est ensuite récupéré après 30 minutes de centrifugation (10000 rpm, 10 °C) et conservé à 4 °C.

La production recombinante de dextrane-saccharase chez *Escherichia coli* utilisant les conditions décrites ici atteint environ 30 000 Unités d'activité enzymatique par litre de culture, ce qui permet son utilisation à faible coût dans des procédés de synthèse de polymère.

**Exemple 3 : Méthode de détermination de l'activité enzymatique de l'enzyme DSR-OK.**

Une unité enzymatique glucane-saccharase représente la quantité d'enzyme qui libère une $\mu$mole de fructose par minute, à 30 °C, à partir de 100 g.L$^{-1}$ de saccharose dans 50mM de tampon acétate de sodium, à pH 5,75.

L'activité est déterminée par mesure de la vitesse initiale de production des sucres réducteurs à l'aide de la méthode à l'acide dinitro-salicilique (DNS). Au cours d'une cinétique, 100 $\mu$L de milieu réactionnel sont prélevés et la réaction est stoppée par ajout d'un volume équivalent de DNS. Les échantillons sont ensuite chauffés 5 min à 95 °C, refroidis dans la glace, dilués au demi dans de l'eau, et l'absorbance est lue à 540 nm. Une gamme étalon de 0 à 2 g.L$^{-1}$ de fructose permet d'établir le lien entre valeur d'absorbance et concentration en sucres réducteurs.

**Exemple 4 : Détermination des conditions optimales de travail de l'enzyme DSR-OK.**

La valeur de la température optimale a été déterminée en mesurant l'activité de l'extrait enzymatique brut, à différentes températures (entre 23 et 40 °C) à partir de 100 g.L$^{-1}$ de saccharose dans 50 mM de tampon acétate de sodium, à pH 5,75.

L'enzyme dextrane-saccharase DSR-OK recombinante a une température optimale de 30°C.

L'effet du pH sur l'activité enzymatique de l'extrait enzymatique brut est mesuré à 30°C à partir de 100 g.L$^{-1}$ de saccharose, dans 50 mM de tampon citrate phosphate, pour des valeurs de pH comprises entre 3,5 et 8 (intervalle de 0,5).

L'enzyme dextrane-saccharase DSR-OK recombinante a un pH optimal compris entre 5 et 5.8.

**Exemple 5 - Détermination des rendements de production et de l'activité polymérase de l'enzyme dextrane-saccharase DSR-OK recombinante.**

Les rendements de production sont déterminés par chromatographie d'échange d'anion (HPAEC-PAD, High Performance Anion Exchange Chromatography with Pulsed Amperometric Detection), pour une réaction enzymatique à 1 U.mL$^{-1}$ de DSR-OK, à partir de 100 g.L$^{-1}$ de saccharose dans 50 mM de tampon acétate de sodium, pH 5,75. Les sucres, glucose, fructose, leucrose et saccharose sont séparés sur colonne Dionex CarboPac PA-100 par un gradient d'acétate de sodium de 6 à 500 mM en 36 min, contenant 150 mM de soude. Des gammes étalons de 5, 10, 15 et 20 mg.kg$^{-1}$ de ces sucres sont réalisées et permettent leur quantification.

Les rendements de production, soit la part de glucose issu du saccharose, incorporée dans la formation de glucose libre, de leucrose et de dextrane sont calculés comme suit :

$$\%G \; glucose = \frac{([Glucose]tf - [Glucose]t0) \times 342}{([Sucrose]t0 - [Sucrose]tf) \times 180} \times 100$$

$$\%G \; leucrose = \frac{([Leucrose]tf - [Leucrose]t0)}{([Sucrose]t0 - [Sucrose]tf)} \times 100$$

L'analyse des produits de la réaction par chromatographie d'exclusion de taille (HPSEC) montrent qu'il n'y a synthèse que de glucose, leucrose et de dextrane de très haute masse moléculaire. Suite à des problèmes récurrents de colmatage des colonnes pendant l'analyse avec ce polymère, la quantification directe du dextrane de très haute masse moléculaire n'est pas réalisable. Le pourcentage d'unités glucosyle incorporé dans la synthèse de dextrane a été déduit des analyses HPAEC-PAD comme ceci :

$$\%G\ dextrane = 100\% - (\%G\ glucose + \%G\ leucrose)$$

Il a ici été mis en évidence que l'enzyme est une excellente polymérase. En effet, les analyses chromatographiques (HPAEC-PAD et HPSEC-RI) réalisées suite à une synthèse de dextrane à partir de 100 g.L$^{-1}$ de saccharose montrent qu'environ 89% (plus précisément, 86% $\pm$ 5%) des unités glucosyle issues du substrat sont utilisées pour la production du polymère dans les conditions standards de production (100 g.L$^{-1}$ de saccharose, à 30°C et à pH 5,75).

Seuls 2% et 9% de ces unités sont perdus par incorporation dans la synthèse de glucose libre et de leucrose, respectivement.

**Exemple 6 - Etude de la stabilité de l'enzyme recombinante DSR-OK.**

Afin d'étudier la stabilité de DSR-OK à 30 °C, l'enzyme (purifiée ou non) est placée à 30°C, initialement à 40 U.mL$^{-1}$, dans 50 mM de tampon acétate de sodium, pH 5,75. L'activité résiduelle est mesurée, à intervalle régulier, à partir de 100 g.L$^{-1}$ de saccharose, dans 50 mM de tampon acétate de sodium, pH 5,75, jusqu'à dénaturation complète de la protéine. Le temps de demi-vie correspond au temps auquel l'enzyme a perdu la moitié de son activité enzymatique initiale.

Comme visible à la figure 2, le temps de demi-vie de l'enzyme DSR-OK à 30°C est de 111h $\pm$ 10h lors d'une caractérisation en milieu brut (non purifiée). L'enzyme DSR-OK est donc très stable, ce qui est une propriété particulièrement intéressante pour une glucane-saccharase de la famille 70 des glycoside-hydrolases.

En comparaison, DSR-S vardel Δ4N, autre glucane-saccharase recombinante présente un temps de demi-vie de seulement 24h dans les mêmes conditions.

**Exemple 7 - Purification de l'enzyme recombinante DSR-OK par chromatographie d'affinité.**

L'enzyme recombinante DSR-OK produite chez *Escherichia coli* est fusionnée à deux étiquettes de purification 6xHis et Strep Tag II aux extrémités N et C-terminales respectivement, afin de permettre une purification par affinité. Une double purification sur colonne StrepTactin (affinité pour le tag Strep Tag II), s'est avérée être la méthode la plus efficace et d'excellente qualité.

Les purifications enzymatiques sont réalisées sur le système ÄKTAxpress de GE Healthcare, dans une enceinte à 8°C. 10 mL d'extrait enzymatique contenant la protéine DSR-Ok tagguée sont injectés dans une colonne Strep Tactin Sepharose High PerformanceTM de 5 mL (GE Healthcare), pré-équilibrée avec du tampon PBS 1X, 280 mM NaCL pH 7,4. Après une heure de fixation en circuit fermé, l'élution est réalisée, à 4 mL.min-1, par un gradient de D-desthiobiotine de 0,05 à 2,5 mM dans du tampon de fixation (PBS 1X, 280 mM NaCl, pH 7,4) sur 20 volumes de colonnes. La fraction purifiée est dessalée sur une colonne 10 DG (Biorad) pré-équilibrée avec 50 mM de tampon acétate de sodium, pH 5,75, 0,05 g.L$^{-1}$ CaCl$_2$, 0,1% Tween80. Afin d'améliorer le facteur de purification et la qualité de la préparation enzymatique pure, l'extrait précédemment obtenu est à nouveau purifié selon ces mêmes conditions.

La concentration en protéines est mesurée à 280 nm au spectrophotomètre Nanodrop 1000 3.7.1 de Thermo Scientific, en fixant le coefficient d'extinction molaire de l'enzyme DSR-OK à 224160 M$^{-1}$.cm$^{-1}$ et son poids moléculaire à 165124 Da (prédit par le programme ProtParam d'Expasy disponible en ligne).

**Exemple 8 - Détermination des paramètres cinétiques de l'enzyme dextrane-saccharase DSR-OK recombinante.**

Les paramètres cinétiques (Vm, Km et Kcat) ont été déterminés à 30 °C, dans 50 mM de tampon acétate de sodium, pH 5,75, supplémenté avec 250 mg.L$^{-1}$ de BSA. Les vitesses initiales sont mesurées pour des concentrations en saccharose variant de 2 à 600 mM, utilisant l'enzyme purifiée à 1 U.mL$^{-1}$ final. Des prélèvements sont effectués à intervalles réguliers, et la réaction enzymatique est arrêtée par chauffage à 95 °C pendant 5 min.

Les échantillons sont ensuite analysés par chromatographie liquide à haute performance (HPLC) sur colonne Aminex Biorad HPX-87K (300$\times$7.8 mm, Biorad). La température du four de la colonne est maintenue à 65 °C et de l'eau ultra-pure est utilisée comme éluant à un débit de 0,6 mL.min$^{-1}$. Des gammes étalons de 5, 10, 15 et 20 g.kg$^{-1}$ de saccharose, leucrose, glucose et fructose sont réalisées pour permettre la quantification des différents sucres. La détection se fait par réfractométrie.

DSR-OK étant une enzyme michaelienne, les paramètres cinétiques, Vm et Km sont déterminés à partir de la représentation graphique de Lineweaver et Burk, selon l'équation $\frac{1}{vi} = \left(\frac{Km}{Vmax} \times \frac{1}{[S]}\right) + \frac{1}{Vmax}$ où [S] représente la concentration initiale en saccharose et vi, la vitesse initiale.

Les paramètres cinétiques de l'enzyme ont été déterminés dans 50 mM de tampon acétate de sodium pH 5.75, en présence de BSA (250 mg.L$^{-1}$) et à 30°C.

Il a ainsi été mis en évidence que DSR-OK, qui suit un mécanisme Michaelien avec une inhibition par excès de substrat, est un catalyseur très performant avec une constante d'affinité Km de 9 mM $\pm$ 1 mM et une constante catalytique Kcat de 550 s$^{-1}$ (figures 3 et 4).

Ces valeurs sont voisines de celles de la dextrane-saccharase DSR-S vardel Δ4N qui est une des enzymes les plus performantes parmi les glucane-saccharases caractérisées de la famille 70 des glycoside-hydrolases (Km de 7.5 mM et Kcat de 584 s$^{-1}$).

DSR-OK se distingue cependant de la dextrane-saccharase DSR-S vardel Δ4N en étant beaucoup moins inhibée par un excès de substrat. En effet, comme visible à la figure 5, sa constante d'inhibition ne s'élève qu'à 1M contre 0,326M pour DSR-S vardel Δ4N.

## Exemple 9 - Synthèse de dextrane par la dextrane-saccharase DSR-OK recombinante.

Les synthèses de dextrane sont réalisées à partir de concentrations variables en saccharose (généralement 100 g.L$^{-1}$), à 30 °C dans 50 mM de tampon acétate de sodium, pH 5,75 et en utilisant 1 U.mL$^{-1}$ d'enzyme sur une durée de 15 h. Pour la plupart des analyses, le polymère a été purifié par deux précipitations à l'éthanol 50%, suivi de deux lavages et d'une re-suspension dans de l'eau ultra-pure avant d'être lyophilisé.

## Exemple 10 - Analyse de la nature des liaisons du dextrane produit par la dextrane-saccharase DSR-OK recombinante.

Après lyophilisation, 20 mg de dextrane purifié sont dilués dans 0,5 mL d'eau deutérée et analysés par RMN du proton avec le spectromètre Bruker Avance (500 MHz). Les spectres sont ensuite traités et interprétés avec le logiciel TOPSPIN 3.0.

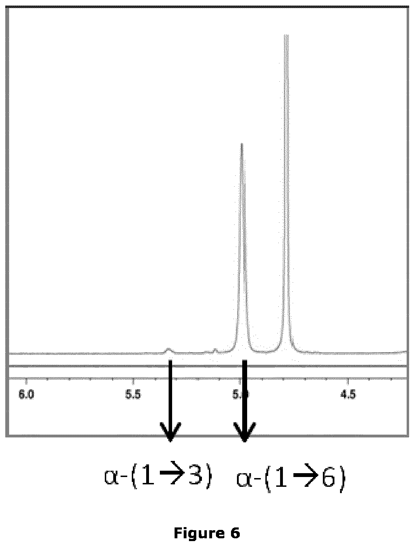

Il a ainsi été mis en évidence par les analyses RMN que le produit synthétisé à partir de 100 g.L$^{-1}$ de saccharose, à 30°C, pH 5.75, est un polymère d'unités glucosyle liées à 97,6% ($\pm$ 0.2%) en α-1,6, et 2,4% en α-1,3, comme le montre la figure 6.

Il s'agit donc d'un dextrane quasi-linéaire, et cela met encore en évidence que la dextrane-saccharase DSR-OK est une dextrane-saccharase très spécifique de la polymérisation par liaisons osidiques de type α-1,6.

## Exemple 11 - Détermination des caractéristiques macromoléculaires du dextrane produit par la dextrane-saccharase DSR-OK recombinante.

La masse molaire moyenne en nombre et en poids, et la structure du dextrane synthétisé par DSR-OK, ont été analysées par AFFFF-MALLS (Asymmetric Flow-Field-Flow-Fractionation - Multi-Angle Laser Light Scattering), à partir des milieux de synthèse bruts selon les conditions de production décrites dans l'exemple 9.

Les échantillons ont été dilués 500 fois, dans de l'eau contenant 0,02% d'azoture de sodium et filtrés sur membrane Durapore de 0,45 μm avant injection (rendement de filtration > 90%). Le mode opératoire est le même que celui utilisé pour caractériser les dextranes de haute masse molaire synthétisés par des variants de DSR Δ4N (9). Ainsi, les échantillons sont injectés à 0,2 mL.min$^{-1}$ sur une durée de 300s avec un flux croisé de 1 mL.min$^{-1}$. Une fois l'injection terminée, un temps de relaxation de 60 secondes est imposé aux échantillons. L'élution est réalisée sous un flux d'entraînement de 0,84 mL.min$^{-1}$, à un flux croisé constant de 0,1 mL.min$^{-1}$ pendant 3125 secondes, à température ambiante. Les valeurs des masses molaires ($M_i$) et celles des rayons de giration ($R_{Gi}$) sont déterminées avec le logiciel ASTRA version 5.3.2.13 (Wyatt Technology).

A chaque intervalle de temps (i), la réponse réfractométrique permet de déterminer la concentration $C_i$. La masse molaire et le rayon de giration sont déterminés par extrapolation de la relation de la diffusion de la lumière à angle nul, à l'aide d'un diagramme de Berry selon :

$$\sqrt{\left(\frac{\mathbf{K_C}}{\mathbf{R_\theta}}\right)} = \sqrt{\frac{\mathbf{1}}{\mathbf{M_i}}\left(\mathbf{1} + \frac{\mathbf{16\pi^2 n^2}}{\mathbf{3\lambda^2}}\mathbf{R_{Gi}^2}\sin^2(\theta/2)\right)}$$

Où, **K** est la constante optique, $\mathbf{R_\theta}$, le rapport de Rayleigh, λ, la longueur d'onde du faisceau du laser incident, **n** l'indice de réfraction de la lumière et θ, l'angle d'observation.

Le logiciel ASTRA calcule directement les masses molaires moyennes en poids ($M_w$) et en nombre ($M_n$), ainsi que le rayon de giration moyen en z ($R_{Gz}$) selon les relations suivantes :

$$M_n = \frac{\sum_i c_i}{\sum_i \frac{c_i}{M_i}}$$

$$M_w = \frac{\sum_i c_i M_i}{\sum_i c_i}$$

$$R_{Gz}^2 = \frac{\sum_i c_i M_i R_{Gi}^2}{\sum_i c_i M_i}$$

Le rapport $M_w/M_n$ représente l'indice de dispersité, $D_i$.

La densité ($d_{Gapp}$) est calculée à partir de l'équation suivante :

$$d_{Gapp} = \frac{M_w}{\left(\frac{4\pi}{3}\right) \times R_{Gw}^3}$$

où $R_{Gw}$ représente le rayon de giration moyen en poids.

Le coefficient hydrodynamique, $\upsilon_G$, est déterminé à partir de la représentation graphique du rayon de giration ($R_{Gi}$) en fonction de la masse molaire ($M_i$) et selon l'équation :

$$R_{Gi} = K_G \cdot M_i^{\upsilon_G}$$

où $K_G$ est une constante.

La valeur du paramètre de branchement $g_M$ est calculée selon la relation suivante :

$$g_M = \frac{\overline{R}_{Gw(br)}^2}{\overline{R}_{Gw\,(lin)}^2}$$

où $R_{Gw(br)}$ et $R_{Gw(lin)}$ représentent les rayons de giration moyen en poids du polymère branché de son équivalent linéaire de même nature chimique et de même masse molaire.

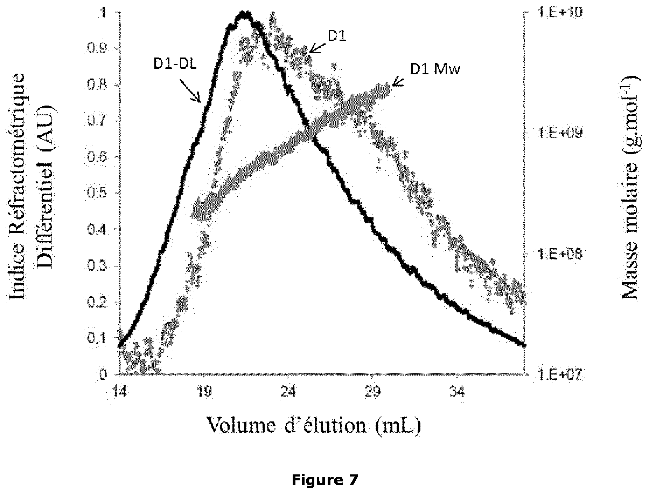

Il a ainsi été mis en évidence par l'analyse des caractéristiques macromoléculaires que le dextrane présente une très haute masse molaire moyenne en poids $M_w$ d'environ $1,01.10^9$ g.mol$^{-1}$ ($\pm$ $0,3.10^9$ g.mol$^{-1}$), comme visible à la figure 7.

La masse molaire moyenne en nombre est également élevée, à savoir $M_n = 5.5.10^8$ g.mol$^{-1}$ ($\pm$ $1.6.10^8$ g.mol$^{-1}$).

L'indice de dispersité $D_i$ du dextrane produit par DSR-OK est donc d'environ 1,8, ce qui représente un indice très faible par rapport aux dextranes produits jusqu'à présent.

Par exemple, en comparaison du dextrane produit par la souche historique *L. mesenteroides* NRRL B-512F, et du dextrane produit par l'enzyme recombinante DSR-S vardel Δ4N, le dextrane produit par la dextrane-saccharase DSR-OK est de taille plus élevée, et est nettement moins polydisperse que le dextrane natif produit par la souche *L. mesenteroides* NRRL B-512F.

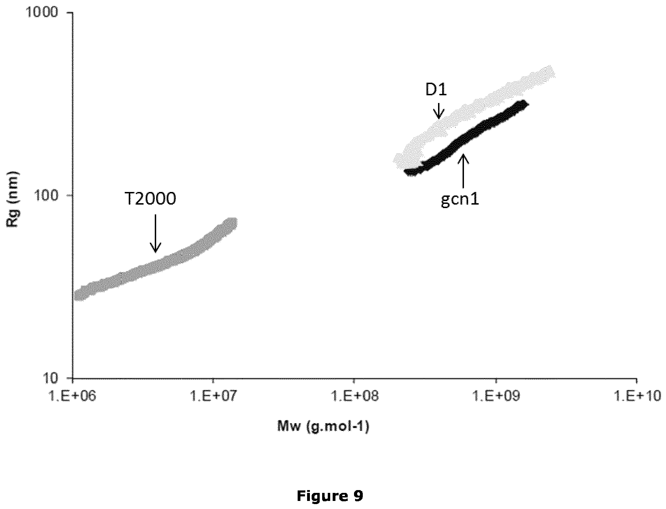

Le rayon de giration du polymère est aussi extrêmement élevé, de l'ordre de 370nm (Figure 9). Le coefficient hydrodynamique, soit la pente du graphique représentant le rayon de giration en fonction de la masse molaire (Figure 9) est de 0.48. Cette valeur montre qu'il s'agit d'un polymère quasi-linéaire, très peu branché.

Le tableau 2 reprend les principales caractéristiques macromoléculaires du dextrane produit par DSR-OK recombinante.

*Tableau 2*

| Caractéristique | Valeur |
|---|---|
| $M_w$ (masse molaire moyenne en poids) | $1.10^9$ g.mol$^{-1}$ $\pm$ $0.3.10^9$ g.mol$^{-1}$ |
| $M_n$ (masse molaire moyenne en nombre) | $5.5.10^8$ g.mol$^{-1}$ $\pm$ $1.6.10^8$ g.mol$^{-1}$ |
| $D_i=M_w/M_n$ (indice de dispersité) | $1.8 \pm 0.3$ |
| $R_{Gz}$ (rayon de giration moyen en z) | 370 nm $\pm$ 18.5 nm |
| $d_{Gapp}$ (densité apparente) | 8.2 g.mol$^{-1}\cdot$nm$^3$ $\pm$ 2.2 g.mol$^{-1}\cdot$nm$^3$ |
| $\upsilon_G$ (coefficient hydrodynamique) | $0.48 \pm 0.02$ |
| $g_M$ (paramètre de branchement moyen) | $0.0158 \pm 0.004$ |

**Exemple 12 : Analyse du comportement rhéologique du dextrane produit par la dextrane-saccharase DSR-OK recombinante.**

*Courbe d'écoulement et détermination du seuil d'écoulement du dextrane dans son milieu brut de synthèse à partir de différentes concentrations en saccharose.*

Les synthèses sont réalisées dans un volume total de 20 mL, à partir de différentes concentrations initiales en saccharose (50, 75, 100 et 150 g.L$^{-1}$), dans 50 mM de tampon acétate de sodium, pH 5,75, à 1 U.mL$^{-1}$ d'enzyme. La température et l'agitation sont fixées à 30°C et à 60 rpm. La réaction enzymatique est réalisée sur une durée de 15 heures.

Les analyses rhéologiques du dextrane (dans son milieu brut de synthèse) sont réalisées sur le rhéomètre HAAKE MARS III de Thermo Scientific, piloté par le logiciel HAAKE RheoWin 4. Toutes les mesures sont déterminées en utilisant une géométrie plan-plan (35 mm de diamètre), à un entrefer de 0.5 mm, à 20 °C (peltier MTMC MarsIII) et à pression atmosphérique.

Les courbes d'écoulement sont obtenues en faisant varier le gradient de vitesse de $10^{-1}$ à $10^3$ s$^{-1}$. Les valeurs de seuil d'écoulement sont déterminées à partir de la représentation graphique traçant les valeurs de la contrainte en fonction du gradient de vitesse. Le seuil d'écoulement est déterminé par la contrainte obtenue à vitesse de cisaillement la plus faible (tendant vers un plateau)

*Courbe de viscosité et mesures visco-élastiques du dextrane purifié*

Le polymère, purifié par deux précipitations à l'éthanol et lyophilisé, est re-suspendu à 50 g.L$^{-1}$ dans de l'eau distillée et solubilisé pendant une nuit à 25 °C, avec une agitation douce. Les propriétés rhéologiques sont mesurées avec un rhéomètre à déformation imposée (ARES, TA.) en utilisant une géométrie cône-plan (diamètre de 5 cm, angle du cône de 0,05 rad) à 20 °C et à pression atmosphérique. La courbe d'écoulement est déterminée en faisant varier le gradient de vitesse de $10^{-2}$ à $10^2$ s$^{-1}$. Les spectres mécaniques (modules G' et G" en fonction de la fréquence) sont déterminés en régime dynamique, en faisant varier la fréquence de $10^{-2}$ à $10^2$ rad.s$^{-1}$, à une amplitude de déformation imposée située dans le domaine de viscoélasticité linéaire.

Il est observé que le polymère dextrane est très visqueux à l'oeil nu et qu'il est nécessaire d'appliquer une force pour qu'il s'écoule.

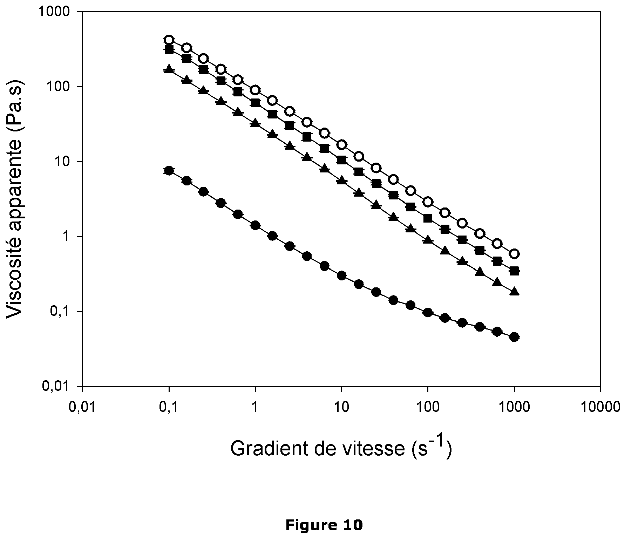

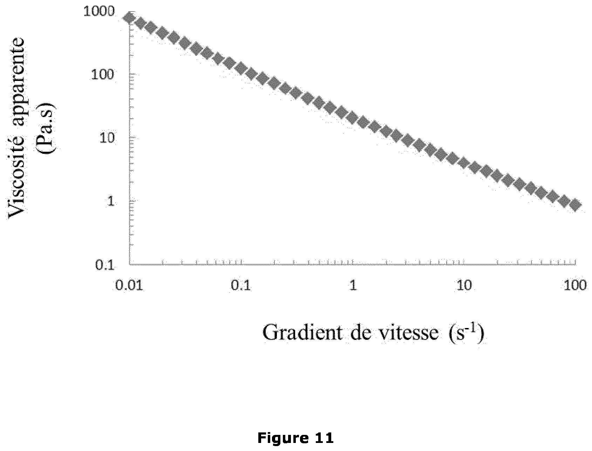

Les courbes d'écoulement déterminées à partir du milieu brut de synthèse pour différentes concentrations initiales en saccharose (50, 75, 100, et 150 g.L$^{-1}$) sont présentées à la Figure 10 et les courbes d'écoulement déterminées à partir de polymère purifié par précipitation alcoolique et préparé à une concentration de 5% (poids/volume) dans de l'eau distillée à la Figure 11.

Dans les deux cas, la viscosité dynamique de la solution est très élevée pour de faibles vitesses de cisaillement (de l'ordre de 100 Pa.s à 0.1s$^{-1}$, Figures 10 et 11). Le polymère adopte un comportement de type rhéofluidifiant ou pseudo-plastique, c'est-à-dire que sa viscosité dynamique diminue lorsque le gradient de vitesse (ou vitesse de cisaillement) augmente.

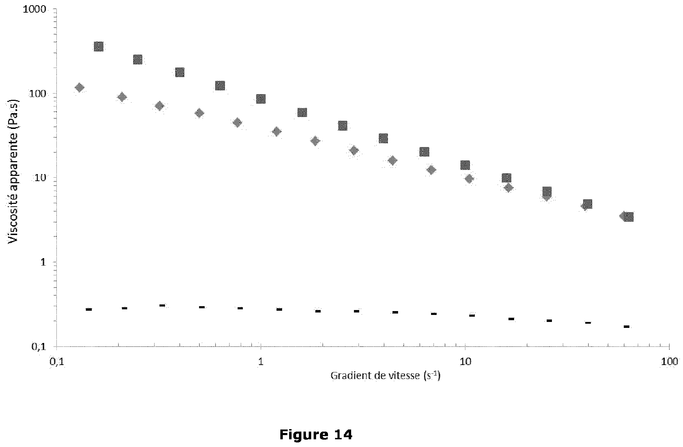

Comme visible à la figure 14, le dextrane produit par la dextrane-saccharase DSR-OK a une viscosité environ 500 fois plus importante que le dextrane natif produit par *L. mesenteroides* NRRL B-512F pour de faibles de contraintes de cisaillement appliquées. En outre, le dextrane natif présente un comportement newtonien contrairement aux dextranes produits par DSR-OK ou DSR-S vardel Δ4N qui sont rhéofluidifiants.

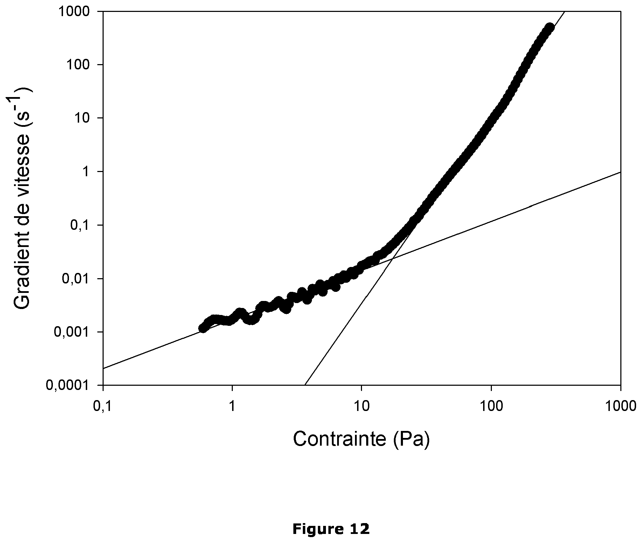

De plus, le dextrane en solution présente un seuil d'écoulement. Ainsi, des seuils d'écoulement de 26 Pa et 38 Pa ont été mesurés pour le dextrane dans le milieu de synthèse brut à partir de 100g.L$^{-1}$ et 200g.L$^{-1}$ de saccharose respectivement (Figure 12).

En comparaison, le dextrane du milieu de synthèse produit par l'enzyme recombinante DSR-S vardel ∆4N à partir de 100g.L$^{-1}$ présente un seuil d'écoulement de 12 Pa.

De faibles concentrations en dextrane pourraient être utilisées pour des produits nécessitant un seuil d'écoulement, par exemple en remplacement de solution de gomme de xanthane ou de solution de guar, utilisés comme agents épaississants et ayant des seuils d'écoulement à 10g.L$^{-1}$ de 7 et 4 Pa, respectivement. Le dextrane conforme à la présente invention peut donc être utilisé dans des produits de type dentifrice, sauces etc.

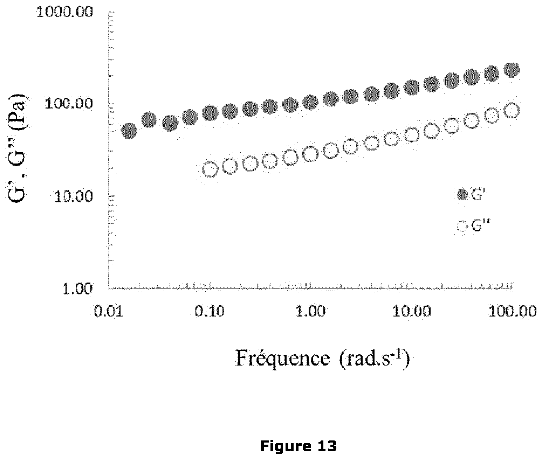

Les tests de balayages en fréquences, en conditions oscillatoires, indiquent par ailleurs que le polymère est un gel faible. En effet, comme visible à la figure 13, le module élastique G' est supérieur au module visqueux G" dans la plage de fréquences testées, de 10$^{-2}$ à 10$^{2}$ rad.s$^{-1}$.

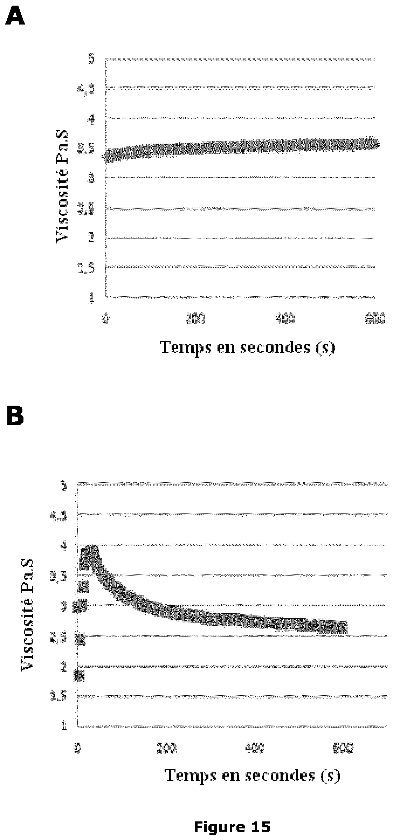

Enfin, il est remarqué que la viscosité du polymère produit par DSR-OK reste stable lorsqu'on applique des contraintes de cisaillement constantes de longue durée, tandis que la viscosité du polymère produit par DSR-S vadel ∆4N diminue dans les mêmes conditions (Figure 15). Le dextrane conforme à la présente invention peut donc être utilisé dans des procédés industriels pour lesquels une agitation permanente est nécessaire.

**Exemple 13 : Détermination de la température de transition vitreuse et de la teneur en eau du dextrane produit par la dextrane-saccharase DSR-OK recombinante.**

Le polymère purifié par deux précipitations à l'éthanol et lyophilisé est équilibré à 57% et à 43% d'humidité relative en le plaçant sous vide en présence de solutions de NaBr et de $K_2CO_3$ saturées à température ambiante pendant une semaine. Les températures de transition vitreuse ($T_g$) sont déterminées au moyen d'un système d'analyse enthalpique différentielle, la DSC Q100 (TA Instruments, France). L'appareil est calibré avec de l'indium. Les mesures sont effectuées sur 2 à 30 mg d'échantillon en utilisant des capsules scellées en aluminium (TA Instruments, Guyancourt, France), chauffées de 0 à 120 °C à 3 °C.min$^{-1}$. Deux scans de chauffage ont été réalisés, espacés par une phase de refroidissement jusqu'à 0 °C à 10 °C.min$^{-1}$, permettant de se prévenir de toute signature due au vieillissement de l'échantillon. Une capsule vide est utilisée comme référence. La température de transition vitreuse ($T_g$) est prise au point d'inflexion du changement de capacité calorifique.

La teneur en eau est déterminée après chaque mesure calorimétrique par analyse thermogravimétrique (TGA) en utilisant un système TGA2050 (T.A. Instruments, New Castle, DE, U.S.A.). La teneur en eau correspond à la perte massique lorsque l'on chauffe l'échantillon jusqu'à 130 °C à 10 °C.min$^{-1}$ puis qu'on le maintien à cette température pendant 40 min.

Les analyses de DSC (Differential Scanning Calorimetry) ont ainsi permis de déterminer des températures de transition vitreuse de 95°C ($\pm$1°C) pour un teneur en eau de 6,6%, et de 25°C ($\pm$1°C) pour une teneur en eau de 12,9%.

Pour une teneur en eau d'environ 13%, le polymère présentera donc un état caoutchouteux aux températures supérieures à 25°C et sera considéré comme « cassant » en-dessous de 25°C.

**REFERENCES**

(1) Characterization of *Leuconostoc mesenteroides* NRRL B-512F dextransucrase (DSRS) and identification of amino-acid residues playing a key role in enzyme activity, Monchois et al., Applied Microbiology and Biotechnology, October 1997, vol 48, Issue 4, 465-472

(2) High-level production and purification of a fully active recombinant dextransucrase from Leuconostoc mesenteroides NRRL B-512F, Moulis et al, FEMS Microbiology Letters, August 2006, vol 261, Issue 2, 203-210

(3) Functional Divergence in the Genus Oenococcus as Predicted by Genome Sequencing of the Newly-Described Species, Oenococcus kitaharae, Borneman et al, PloS ONE, January 2012, vol. 7, Issue 1

(4) Oenococcus kitaharae sp. nov., a non-acidophilic and non-malolactic-fermenting oenococcus isolated from a composting distilled shochu residue, Akihito Endo and Sanae Okada, International Journal of Systematic and Evolutionary Microbiology (2006), 56, 2345-2348

(5) Glucansucrases: Three-dimensional structures, reactions, mechanism, alpha-glucan analysis and their implications in biotechnology and food applications Lemhuis et al., Journal of Biotechnology, January 2013, vol 163, Issue 2, 250-272

(6) Dextran: effect of process parameters on production, purification and molecular weight and recent applications, Vettori et al., Diálogos & Ciência, ISSN 1678-0493, no 31, septembre 2012

(7) Functional Polymers Based on Dextran, Thomas Heinze, Tim Liebert, Brigitte Heublein, Stephanie Hornig, Adv Polym Sci (2006) 205: 199-291, DOI 10.1007/12_100.

(8) Structure and macromolecular properties of Weissella confusa and Leuconostoc citreum dextrans with a potential application in sourdough, Ndegwa Henry Maina, 1st June 2012, University of Helsinki, Department of Food and Environmental Sciences, Chemistry and Biochemistry Division.

(9) Structure and Property Engineering of alpha-D-Glucans Synthesized by Dextransucrase Mutants, Irague et al, BioMacromolecules, 2012, vol 13, Issue 1, 187-195.

(10) A method for determination of invertase activity, Sumner & Howell, Journal of biological chemistry, 1935, vol 108, Issue 51

(11) Online Determination of Structural Properties and Observation of Deviations from Power Law Behavior, Rolland-Sabaté et al, Biomacromolecules, 2008, vol 9, 1719-1730.

**Revendications**

1. Dextranes **caractérisés en ce qu'**ils présentent entre 95% et 99%, de préférence entre 97% et 98%, de préférence encore entre 97,4% et 97,6%, de liaisons glucosidiques $\alpha$-1,6, une masse molaire moyenne en poids $M_w$ au moins égale à $0,7.10^9$ g.mol$^{-1}$, et un indice de dispersité $D_i$ compris entre 1,3 et 3.

2. Dextranes selon la revendication 1, **caractérisé en ce qu'**ils présentent une masse molaire moyenne en poids $M_w$ au moins égale à $1.10^9$ g.mol$^{-1}$.

3. Dextrane-saccharase ayant au moins 80%, de préférence 85%, de préférence encore 90%, de préférence encore 95%, de préférence encore 98% d'identité avec la séquence allant des positions 359 à 1038 de la séquence d'acides aminés SEQ ID NO : 1, la dextrane-saccharase n'ayant pas pour séquence d'acides aminés la séquence SEQ ID NO : 1.

4. Procédé de fabrication de dextranes selon la revendication 1 ou 2 comprenant la réaction d'une dextrane-saccharase ayant pour séquence d'acides aminés la séquence SEQ ID NO : 1, ou au moins 80%, de préférence 85%, de préférence encore 90%, de préférence encore 95%, de préférence encore 98% d'identité avec la séquence allant des positions 359 à 1038 de la séquence d'acides aminés SEQ ID NO : 1 avec du saccharose.

5. Procédé de fabrication selon la revendication 4, **caractérisé en ce que** la réaction est conduite à une température comprise entre 20°C et 40°C, de préférence comprise entre 25°C et 35°C, de préférence encore comprise entre 30°C et 33°C.

6. Procédé de fabrication selon la revendication 4 ou 5, **caractérisé en ce que** la concentration en saccharose est comprise entre environ 50 et 200 g.L$^{-1}$, de préférence entre environ 50 et 150 g.L$^{-1}$.

7. Procédé de fabrication selon l'une quelconque des revendications 4 à 6, **caractérisé en ce que** le pH lors de la réaction est compris entre environ 5 et 6,5, de préférence entre 5 et 6, de préférence encore entre 5 et 5,8.

8. Composition cosmétique ou alimentaire comprenant des dextranes selon la revendication 1 ou 2 et au moins un support pharmaceutiquement acceptable ou alimentaire.

9. Utilisation de dextranes selon la revendication 1 ou 2 en tant qu'agent épaississant, gélifiant et/ou texturant.

**Patentansprüche**

1. Dextrane, **dadurch gekennzeichnet, dass** sie zwischen 95% und 99%, bevorzugt zwischen 97% und 98%, stärker bevorzugt zwischen 97,4% und 97,6%, $\alpha$-1,6-Glucosidbindungen, eine gewichtsmittlere Molmasse $M_w$ von mindestens gleich $0,7.10^9$ g.mol$^{-1}$ und einen Dispersitätsindex $D_i$ zwischen 1,3 und 3 aufweisen.

2. Dextrane nach Anspruch 1, **dadurch gekennzeichnet, dass** sie eine gewichtsmittlere Molmasse $M_w$ von mindestens gleich $1.10^9$ g.mol$^{-1}$ aufweisen.

3. Dextran-Saccharase, die mindestens 80%, bevorzugt 85%, stärker bevorzugt 90%, stärker bevorzugt 95%, stärker bevorzugt 98% Identität mit der Sequenz, die von den Positionen 359 bis 1038 der Aminosäuresequenz SEQ ID NO: 1 reicht, aufweist, wobei die Dextran-Saccharase als Aminosäuresequenz nicht die Sequenz SEQ ID NO: 1 aufweist.

4. Verfahren zur Herstellung von Dextranen nach Anspruch 1 oder 2, umfassend die Reaktion einer Dextran-Saccharase, die als Aminosäuresequenz die Sequenz SEQ ID NO: 1 oder mindestens 80%, bevorzugt 85%, stärker bevorzugt 90%, stärker bevorzugt 95%, stärker bevorzugt 98% Identität mit der Sequenz, die von den Positionen 359 bis 1038 der Aminosäuresequenz SEQ ID NO: 1 reicht, aufweist, mit Saccharose.

5. Verfahren zur Herstellung nach Anspruch 4, **dadurch gekennzeichnet, dass** die Reaktion bei einer Temperatur zwischen 20 °C und 40 °C, bevorzugt zwischen 25 °C und 35 °C, stärker bevorzugt zwischen 30 °C und 33 °C durchgeführt wird.

6. Verfahren zur Herstellung nach Anspruch 4 oder 5, **dadurch gekennzeichnet, dass** die Saccharosekonzentration zwischen etwa 50 und 200 g.L$^{-1}$, bevorzugt zwischen etwa 50 und 150 g.L$^{-1}$ liegt.

7. Verfahren zur Herstellung nach einem der Ansprüche 4 bis 6, **dadurch gekennzeichnet, dass** der pH-Wert während der Reaktion zwischen etwa 5 und 6,5, bevorzugt zwischen 5 und 6, stärker bevorzugt zwischen 5 und 5,8 liegt.

8. Kosmetik- oder Lebensmittelzusammensetzung, umfassend Dextrane nach Anspruch 1 oder 2 und mindestens einen pharmazeutisch akzeptablen Träger oder einen Lebensmittelträger.

9. Verwendung von Dextranen nach Anspruch 1 oder 2 als Verdickungs-, Gelierungs- und/oder Texturierungsmittel.

**Claims**

1. Dextrans **characterized in that** they have between 95% and 99%, preferably between 97% and 98%, more preferably between 97.4% and 97.6%, of $\alpha$-1,6 glucosidic bonds, a weight-average molar mass $M_w$ at least equal to $0.7 \times 10^9$ g.mol$^{-1}$, and a dispersity index $D_i$ of between 1.3 and 3.

2. The dextrans as claimed in claim 1, **characterized in that** they have a weight-average molar mass $M_w$ at least equal to $1 \times 10^9$ g.mol$^{-1}$.

3. A dextransucrase having at least 80%, preferably 85%, more preferably 90%, more preferably 95%, more preferably 98% identity with the sequence ranging from positions 359 to 1038 of the amino acid sequence SEQ ID NO: 1, the dextransucrase not having as amino acid sequence the sequence SEQ ID NO: 1.

4. A process for producing dextrans as claimed in claim 1 or 2, comprising the reaction of a dextransucrase having as amino acid sequence the sequence SEQ ID NO: 1, or at least 80%, preferably 85%, more preferably 90%, more preferably 95%, more preferably 98% identity with the sequence ranging from positions 359 to 1038 of the amino acid sequence SEQ ID NO: 1 with sucrose.

5. The production process as claimed in claim 4, **characterized in that** the reaction is carried out at a temperature of between 20°C and 40°C, preferably of between 25°C and 35°C, more preferably of between 30°C and 33°C.

6. The production process as claimed in claim 4 or 5, **characterized in that** the sucrose concentration is preferably between approximately 50 and 200 g.l$^{-1}$, preferably between approximately 50 and 150 g.l$^{-1}$.

7. The production process as claimed in any one of claims 4 to 6, **characterized in that** the pH during the reaction is between approximately 5 and 6.5, preferably between 5 and 6, more preferably between 5 and 5.8.

8. A cosmetic or food composition comprising dextrans as claimed in claim 1 or 2 and at least one pharmaceutically acceptable or food-grade support.

9. The use of dextrans as claimed in claim 1 or 2, as a thickener, gelling agent and/or texturing agent.

Figure 1

**Figure 2**

**Figure 3**

**Figure 4**

**Figure 5**

α-(1→3)   α-(1→6)

**Figure 6**

**Figure 7**

**Figure 8**

**Figure 9**

**Figure 10**

**Figure 11**

**Figure 12**

**Figure 13**

**Figure 14**

**A**

**B**

Figure 15

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2978773 A

- US 6627235 B

**Littérature non-brevet citée dans la description**

- **MONCHOIS et al.** *Applied Microbiology and Biotechnology,* Octobre 1997, vol. 48 (4), 465-472
- **MOULIS et al.** High-level production and purification of a fully active recombinant dextransucrase from Leuconostoc mesenteroides NRRL B-512F. *FEMS Microbiology Letters,* Août 2006, vol. 261 (2), 203-210
- **BORNEMAN et al.** Functional Divergence in the Genus Oenococcus as Predicted by Genome Sequencing of the Newly-Described Species, Oenococcus kitaharae. *PloS ONE,* Janvier 2012, vol. 7 (1
- **AKIHITO ENDO ; SANAE OKADA.** Oenococcus kitaharae sp. nov., a non-acidophilic and non-malolactic-fermenting oenococcus isolated from a composting distilled shochu residue. *International Journal of Systematic and Evolutionary Microbiology,* 2006, vol. 56, 2345-2348
- **LEMHUIS et al.** Glucansucrases: Three-dimensional structures, reactions, mechanism, alpha-glucan analysis and their implications in biotechnology and food applications. *Journal of Biotechnology,* Janvier 2013, vol. 163 (2), 250-272

- **VETTORI et al.** Dextran: effect of process parameters on production, purification and molecular weight and recent applications. *Diálogos & Ciência,* Septembre 2012, ISSN 1678-0493
- **THOMAS HEINZE ; TIM LIEBERT ; BRIGITTE HEUBLEIN ; STEPHANIE HORNIG.** Functional Polymers Based on Dextran. *Adv Polym Sci,* 2006, vol. 205, 199-291
- **NDEGWA HENRY MAINA.** Structure and macromolecular properties of Weissella confusa and Leuconostoc citreum dextrans with a potential application in sourdough. University of Helsinki, Department of Food and Environmental Sciences, Chemistry and Biochemistry Division, 01 Juin 2012
- **IRAGUE et al.** Structure and Property Engineering of alpha-D-Glucans Synthesized by Dextransucrase Mutants. *BioMacromolecules,* 2012, vol. 13 (1), 187-195
- **SUMNER ; HOWELL.** A method for determination of invertase activity. *Journal of biological chemistry,* 1935, vol. 108 (51
- **ROLLAND-SABATÉ et al.** Online Determination of Structural Properties and Observation of Deviations from Power Law Behavior. *Biomacromolecules,* 2008, vol. 9, 1719-1730